(19) Europäisches Patentamt
European Patent Office
Office européen des brevets

(11) EP 0 559 612 B1

(12) # EUROPÄISCHE PATENTSCHRIFT

(45) Veröffentlichungstag und Bekanntmachung des Hinweises auf die Patenterteilung:
**14.08.1996 Patentblatt 1996/33**

(51) Int. Cl.⁶: **C07D 317/22**, A01N 43/28, C07D 317/34, C07D 317/72, C07C 43/295, C07C 49/84, C07C 43/23

(21) Anmeldenummer: **93810125.0**

(22) Anmeldetag: **23.02.1993**

(54) **Dioxolanderivate als Schädlingsbekämpfungsmittel**
Dioxolane derivatives as pesticides
Dérivés de dioxolane comme pesticides

(84) Benannte Vertragsstaaten:
**AT BE CH DE DK ES FR GB GR IE IT LI LU MC NL PT SE**

(30) Priorität: **02.03.1992 CH 643/92**

(43) Veröffentlichungstag der Anmeldung:
**08.09.1993 Patentblatt 1993/36**

(73) Patentinhaber: **CIBA-GEIGY AG**
**4002 Basel (CH)**

(72) Erfinder: **Karrer, Friedrich, Dr.**
**CH-4800 Zofingen (CH)**

(56) Entgegenhaltungen:
**EP-A- 0 276 196** **FR-A- 2 334 680**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Rank Xerox (UK) Business Services
2.13.2/3.4

## Beschreibung

Die Erfindung betrifft Verbindungen der Formel

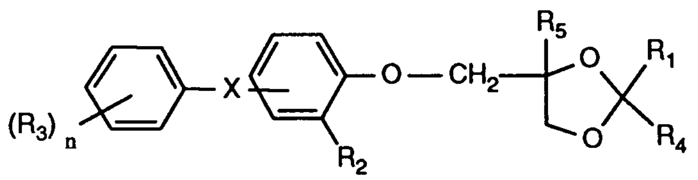

(I),

worin bedeuten:

n 0, 1 oder 2, wobei, wenn n 2 ist, die beiden Reste $R_3$ gleich oder verschieden sind;

entweder $R_1$ $C_1$-$C_6$-Alkyl, Halogen-$C_1$-$C_3$-alkyl, $C_2$-$C_4$-Alkenyl, $C_2$-$C_4$-Alkinyl, $C_1$-$C_3$-Alkoxy oder $C_3$-$C_6$-Cycloalkyl und $R_4$ Wasserstoff oder $C_1$-$C_3$-Alkyl oder $R_1$ und $R_4$, zusammen mit dem Kohlenstoffatom, an das $R_1$ und $R_4$ gebunden sind, einen Ring mit 4, 5 oder 6 Ringgliedern, wobei das Ringgerüst, das gegebenenfalls eine Kohlenstoff-Kohlenstoff-Doppelbindung enthält, entweder nur aus Kohlenstoffatomen oder aus 1 Sauerstoffatom und 3, 4 oder 5 Kohlenstoffatomen aufgebaut ist und wobei der Ring unsubstituiert oder ein- oder zweifach durch gleiches oder verschiedenes $C_1$-$C_3$-Alkyl substituiert ist;

$R_2$ $C_1$-$C_3$-Alkyl, Halogen-$C_1$-$C_3$-alkyl, $C_1$-$C_3$-Alkoxy, Halogen-$C_1$-$C_3$-alkoxy, Fluor, Chlor oder Brom;

$R_3$ $C_1$-$C_3$-Alkyl, Halogen-$C_1$-$C_3$-alkyl, $C_1$-$C_3$-Alkoxy, Halogen-$C_1$-$C_3$-alkoxy, Fluor, Chlor oder Brom;

$R_5$ Wasserstoff oder $C_1$-$C_3$-Alkyl und

X Methylen, O, S oder C(=O),

ein Verfahren zur Herstellung und die Verwendung dieser Verbindungen,

Schädlingsbekämpfungsmittel, deren Wirkstoff aus diesen Verbindungen ausgewählt ist, ein Verfahren zur Herstellung und die Verwendung dieser Mittel und Zwischenprodukte für die Herstellung der Verbindungen der Formel I.

Die vor- und nachstehend verwendeten Allgemeinbegriffe haben, sofern nicht abweichend definiert, die nachfolgend aufgeführten Bedeutungen.

Bei den als Substituenten von Halogenalkyl und Halogenalkoxy in Betracht kommenden Halogenatomen handelt es sich sowohl um Fluor und Chlor als auch um Brom und Iod, wobei Fluor, Chlor und Brom bevorzugt sind.

Kohlenstoffhaltige Gruppen und Verbindungen enthalten, sofern nicht abweichend definiert, jeweils vorzugsweise 1 bis und mit 4, insbesondere 1 oder 2, Kohlenstoffatome.

$C_3$-$C_6$-Cycloalkyl ist Cyclopropyl, Cyclobutyl, Cyclopentyl oder Cyclohexyl.

Alkyl - als Gruppe per se sowie als Strukturelement von anderen Gruppen und Verbindungen, wie von Alkoxy, Halogenalkyl und Halogenalkoxy, - ist, jeweils unter gebührender Berücksichtigung der von Fall zu Fall umfassten Anzahl der in der entsprechenden Gruppe oder Verbindung enthaltenen Kohlenstoffatome, entweder geradkettig oder verzweigt und ist Methyl, Ethyl, Propyl, Isopropyl, Butyl, Isobutyl, sek.-Butyl oder tert.-Butyl oder Pentyl, Hexyl, Octyl oder jeweils ein Isomeres davon. Bevorzugte Alkylgruppen $R_1$ sind $C_1$-$C_3$-Alkylgruppen, insbesondere $C_2$-$C_3$-Alkylgruppen.

Alkenyl und Alkinyl enthalten eine oder mehrere, vorzugsweise nicht mehr als zwei, ungesättigte Kohlenstoff-Kohlenstoff-Bindungen. Beispielhaft genannt seien Vinyl, Allyl, Methallyl, Prop-1-en-1-yl, 2-Methyl-prop-1-en-1-yl, But-2-en-1-yl, Ethinyl, Propargyl, Prop-1-in-1-yl und But-1-in-1-yl.

Halogensubstituierte Gruppen, d. h. Halogenalkyl und Halogenalkoxy, können teilweise halogeniert oder perhalogeniert sein. Beispiele für Halogenalkyl - als Gruppe per se sowie als Strukturelement von anderen Gruppen und Verbindungen, wie von Halogenalkoxy, - sind das ein- bis dreifach durch Fluor, Chlor und/oder Brom substituierte Methyl, wie $CHF_2$ oder $CF_3$; das ein- bis fünffach durch Fluor, Chlor und/oder Brom substituierte Ethyl, wie $CH_2CF_3$, $CF_2CF_3$, $CF_2CCl_3$, $CF_2CHCl_2$, $CF_2CHF_2$, $CF_2CFCl_2$, $CF_2CHBr_2$, $CF_2CHClF$, $CF_2CHBrF$ oder $CClFCHClF$; und das ein- bis siebenfach durch Fluor, Chlor und/oder Brom substituierte Propyl oder Isopropyl, wie $CH_2CHBrCH_2Br$, $CF_2CHFCF_3$, $CH_2CF_2CF_3$ oder $CH(CF_3)_2$.

Bevorzugt sind im Rahmen der Erfindung

(1) Verbindungen der Formel

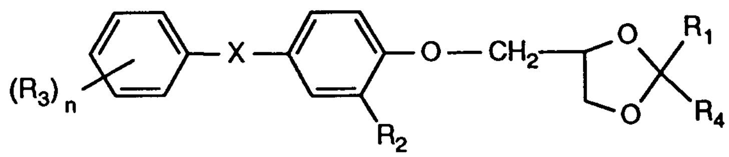

(Ia),

worin bedeuten:

n 0, 1 oder 2, wobei, wenn n 2 ist, die beiden Reste $R_3$ gleich oder verschieden sind;
$R_1$ $C_1$-$C_4$-Alkyl, Vinyl oder Cyclopropyl;
$R_2$ Methyl oder Chlor;
$R_3$ Methyl, Fluor oder Chlor;
$R_4$ Wasserstoff oder Methyl und
X Sauerstoff oder Methylen;

(2) Verbindungen der Formel Ia, worin X Sauerstoff ist, insbesondere solche, worin X Sauerstoff und $R_2$ Chlor bedeuten, ganz besonders solche, worin X Sauerstoff, $R_2$ Chlor und $R_4$ Wasserstoff bedeuten, vor allem solche, worin X Sauerstoff, $R_2$ Chlor, $R_4$ Wasserstoff und $R_1$ $C_1$-$C_4$-Alkyl bedeuten;
(3) Verbindungen der Formel Ia, worin $R_2$ Chlor ist;
(4) Verbindungen der Formel Ia, worin $R_4$ Wasserstoff ist, insbesondere solche, worin $R_4$ Wasserstoff und X Sauerstoff bedeuten;
(5) Verbindungen der Formel Ia, worin $R_1$ $C_1$-$C_4$-Alkyl ist, insbesondere solche, worin $R_1$ $C_1$-$C_4$-Alkyl und X Sauerstoff bedeuten, vor allem solche, worin $R_1$ $C_1$-$C_4$-Alkyl, X Sauerstoff und $R_2$ Chlor bedeuten;
(6) Verbindungen der Formel Ia gemäss den vorstehend definierten Gruppen (1) bis (5), worin n O, 1 oder 2 und $R_3$ Fluor oder Chlor bedeuten, insbesondere solche, worin n 1 oder 2 und $R_3$ Fluor oder Chlor bedeuten, vor allem solche, worin n 1 und $R_3$ Fluor oder Chlor bedeuten;
(7) Verbindungen der Formel Ia gemäss den vorstehend definierten Gruppen (1) bis (6), worin n 1 ist und der Rest $R_3$ in 3- oder 4-Stellung gebunden ist oder n 2 ist und die Reste $R_3$ in 2,4-, 3,4- oder 3,5-Stellung gebunden sind, insbesondere solche, worin n 1 ist und der Rest $R_3$ in 3-Stellung gebunden ist oder n 2 ist und einer der Reste $R_3$ in 3-Stellung gebunden ist, vor allem solche, worin n 1 ist und der Rest $R_3$ in 3-Stellung gebunden ist oder n 2 ist und die Reste $R_3$ in 3,4- oder 3,5-Stellung gebunden sind;
(8) Verbindungen der Formel Ia, worin X Methylen, $R_2$ Chlor und $R_4$ Wasserstoff bedeuten, insbesondere solche, worin X Methylen, $R_2$ Chlor, $R_4$ Wasserstoff und $R_1$ $C_1$-$C_4$-Alkyl bedeuten;
(9) Verbindungen der Formel Ia gemäss der vorstehend definierten Gruppe (8), worin n und $R_3$ die in den vorstehend definierten Gruppen (6) und (7) angegebenen Bedeutungen haben;
(10) Verbindungen der Formel I, worin $R_5$ Wasserstoff ist;
(11) Verbindungen der Formel I, worin die $(R_3)_nC_6H_{5-n}X$-Gruppe in para-Stellung zu $R_2$ oder zur Dioxolanylmethoxygruppe, insbesondere in para-Stellung zur Dioxolanylmethoxygruppe, gebunden ist;
(12) Verbindungen der Formel I, worin die beiden Reste $R_1$ und $R_4$ nicht unter einander verknüpft sind.

Besonders bevorzugt sind im Rahmen der Erfindung die in den Beispielen H3 und H4 genannten Verbindungen der Formel I.

Namentlich bevorzugt sind im Rahmen der Erfindung die folgenden Verbindungen der Formel I:

4-(2-Chlor-4-phenoxy-phenoxymethyl)-2-ethyl-1,3-dioxolan;
4-(2-Chlor-4-phenoxy-phenoxymethyl)-2-propyl-1,3-dioxolan;
4-(2-Chlor-4-phenoxy-phenoxymethyl)-2-isopropyl-1,3-dioxolan;
4-(2-Chlor-4-phenoxy-phenoxymethyl)-2-cyclopropyl-1,3-dioxolan;
4-[2-Chlor-4-(3-fluorphenoxy)-phenoxymethyl]-2-ethyl-1,3-dioxolan;
4-[2-Chlor-4-(3-fluorphenoxy)-phenoxymethyl]-2-propyl-1,3-dioxolan;
4-[2-Chlor-4-(3-fluorphenoxy)-phenoxymethyl]-2-isopropyl-1,3-dioxolan;
4-[2-Chlor-4-(3-fluorphenoxy)-phenoxymethyl]-2-cyclopropyl-1,3-dioxolan;
4-[2-Chlor-4-(3-chlorphenoxy)-phenoxymethyl]-2-ethyl-1,3-dioxolan;
4-[2-Chlor-4-(3-chlorphenoxy)-phenoxymethyl]-2-propyl-1,3-dioxolan;
4-[2-Chlor-4-(3-chlorphenoxy)-phenoxymethyl]-2-isopropyl-1,3-dioxolan;
4-[2-Chlor-4-(3,5-difluorphenoxy)-phenoxymethyl]-2-ethyl-1,3-dioxolan;
4-[2-Chlor-4-(3,5-difluorphenoxy)-phenoxymethyl]-2-propyl-1,3-dioxolan;
4-[2-Chlor-4-(3,5-difluorphenoxy)-phenoxymethyl]-2-isopropyl-1,3-dioxolan;
4-[2-Chlor-4-(3,4-dichlorphenoxy)-phenoxymethyl]-2-ethyl-1,3-dioxolan;

4-[2-Chlor-4-(3-Chlor-4-fluor-phenoxy)-phenoxymethyl]-2-ethyl-1,3-dioxolan;
4-[2-Chlor-4-benzyl-phenoxymethyl)-2-ethyl-1,3-dioxolan;
4-[2-Chlor-4-benzyl-phenoxymethyl)-2-propyl-1,3-dioxolan;
4-[2-Chlor-4-benzyl-phenoxymethyl)-2-isopropyl-1,3-dioxolan;
4-[2-Chlor-4-benzyl-phenoxymethyl)-2-cyclopropyl-1,3-dioxolan;
4-[2-Chlor-4-(3-fluorbenzyl)-phenoxymethyl]-2-ethyl-1,3-dioxolan;
4-[2-Chlor-4-(3-fluorbenzyl)-phenoxymethyl]-2-propyl-1,3-dioxolan;
4-[2-Chlor-4-(3,5-difluorbenzyl)-phenoxymethyl]-2-ethyl-1,3-dioxolan und
4-[2-Chlor-4-(3,4-dichlorbenzyl)-phenoxymethyl]-2-ethyl-1,3-dioxolan.

Als weiterer Gegenstand der Erfindung ist das Verfahren zur Herstellung der Verbindungen der Formel I z. B. dadurch gekennzeichnet, dass man

a) eine Verbindung der Formel

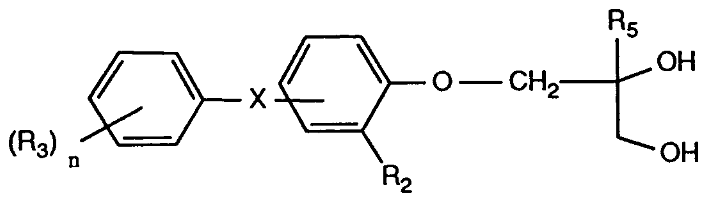

(II)

mit einer Verbindung der Formel

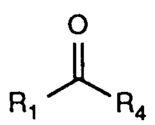

(III),

vorzugsweise in Gegenwart eines sauren Katalysators, umsetzt oder
b) eine Verbindung der Formel

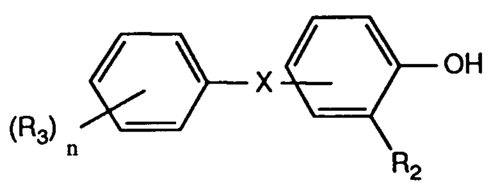

(IV)

mit einer Verbindung der Formel

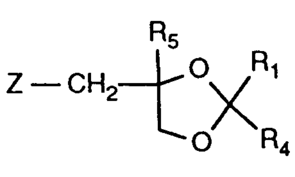

(V),

vorzugsweise in Gegenwart einer Base, umsetzt oder
c) eine Verbindung der Formel II mit einer Verbindung der Formel

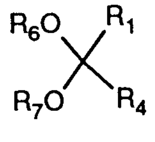

(VI),

vorzugsweise in Gegenwart eines sauren Katalysators, umsetzt, wobei n, $R_1$, $R_2$, $R_3$, $R_4$, $R_5$ und X jeweils die für

die Formel I angegebenen Bedeutungen haben, $R_6$ und $R_7$ entweder, unabhängig voneinander, $C_1$-$C_8$-Alkyl oder zusammen $-(CH_2)_2-$ oder - $(CH_2)_3$-bedeuten und Z Chlor, Brom, Iod, Methansulfonyloxy oder p-Toluolsulfonyloxy bedeutet und jeweils, wenn erwünscht, ein verfahrensgemäss erhältliches Gemisch von Isomeren auftrennt und das gewünschte Isomere isoliert.

Die vor- und nachstehend beschriebenen Umsetzungen werden in an sich bekannter Weise durchgeführt, z.B. in Ab- oder üblicherweise in Anwesenheit eines geeigneten Lösungs-oder Verdünnungsmittels oder eines Gemisches derselben, wobei man je nach Bedarf unter Kühlen, bei Raumtemperatur oder unter Erwärmen, z.B. in einem Temperaturbereich von etwa -80°C bis zur Siedetemperatur des Reaktionsmediums, vorzugsweise von etwa -20°C bis etwa +150°C, beispielsweise bei der Rückflusstemperatur des Reaktionsgemisches, und, falls erforderlich, in einem geschlossenen Gefäss, unter Druck, in einer Inertgasatmosphäre und/oder unter wasserfreien Bedingungen arbeitet. Besonders vorteilhafte Reaktionsbedingungen können den Beispielen entnommen werden.

Das vor- und nachstehend aufgeführte Ausgangsmaterial, das für die Herstellung der Verbindungen I verwendet wird, ist bekannt oder kann nach an sich bekannten Methoden, z.B. gemäss den nachstehenden Angaben, hergestellt werden.

Variante a):

Geeignete saure Katalysatoren sind z. B. Sulfonsäuren, wie Methan- oder p-Toluolsulfonsäure, einschliesslich der sauren Ionenaustauscherharze mit Sulfogruppen, Lewis-Säuren, wie Bortrifluorid-Diethylether- oder -Dimethylether-Komplexe, sowie Mineralsäuren, wie Schwefelsäure oder Phosphorsäure.

Die Reaktionspartner können als solche, d. h. ohne Zusatz eines Lösungs- oder Verdünnungsmittels, z. B. in der Schmelze, miteinander umgesetzt werden. Zumeist ist jedoch der Zusatz eines inerten Lösungs- oder Verdünnungsmittels oder eines Gemisches derselben vorteilhaft. Als Beispiele für solche Lösungs- oder Verdünnungsmittel seien genannt: aromatische, aliphatische und alicyclische Kohlenwasserstoffe und Halogenkohlenwasserstoffe, wie Benzol, Toluol, Xylol, Chlorbenzol, Brombenzol, Petrolether, Hexan, Cyclohexan, Dichlormethan, Trichlormethan, Dichlorethan oder Trichlorethen; Ether, wie Diethylether, tert.-Butylmethylether, Tetrahydrofuran oder Dioxan; Ketone, wie Aceton oder Methylethylketon; Amide, wie N,N-Dimethylformamid, N,N-Dimethylacetamid, N-Methylpyrrolidon oder Hexamethylphosphorsäuretriamid; Nitrile, wie Acetonitril; und Sulfoxide, wie Dimethylsulfoxid.

Die Umsetzung erfolgt vorteilhaft in einem Temperaturbereich von etwa 0°C bis etwa +180°C, bevorzugt von etwa +20°C bis etwa +130°C, in vielen Fällen bei der Rückflusstemperatur des verwendeten Lösungsmittels.

In einer bevorzugten Ausführungsform der Variante a) wird eine Verbindung II bei Rückflusstemperatur, in einem aromatischen Kohlenwasserstoff, vorzugsweise in Toluol, und in Gegenwart einer Sulfonsäure als Katalysator, vorzugsweise in Gegenwart von p-Toluolsulfonsäure, mit einer Verbindung III umgesetzt.

Die Verbindungen II sind bekannt oder können in Analogie zu bekannten Verbindungen hergestellt werden, z. B. durch Umsetzung einer Verbindung der Formel IV mit 2-$R_5$-2,3-Epoxy-propan-1-ol, wobei man in an sich bekannter Weise, vorteilhaft in einem inerten Lösungs- oder Verdünnungsmittel, beispielsweise der vorstehend angegebenen Art, z.B. in einem aromatischen Kohlenwasserstoff, wie Toluol oder Xylol, in Gegenwart eines Katalysators, z.B. eines quartären Ammoniumsalzes, wie in Gegenwart von Tetramethylammoniumchlorid, und bei Raumtemperatur oder erhöhter Temperatur, z.B. in einem Temperaturbereich von etwa 20°C bis etwa 200°C, vorzugsweise von etwa 50°C bis etwa 150°C, arbeitet.

Die Verbindungen der Formel III sind bekannt oder können in Analogie zu bekannten Verbindungen hergestellt werden.

Die Verbindungen IV sind bekannt oder können in Analogie zu bekannten Verbindungen hergestellt werden.

Variante b):

Geeignete Basen zur Erleichterung der HZ-Abspaltung sind z.B. Alkalimetall- oder Erdalkalimetall-hydroxide, -hydride, -amide, -alkanolate, -carbonate, -dialkylamide oder -alkylsilylamide, Alkylamine, Alkylendiamine, gegebenenfalls N-alkylierte, gegebenenfalls ungesättigte, Cycloalkylamine, basische Heterocyclen, Ammoniumhydroxide sowie carbocyclische Amine. Beispielhaft seien Natrium-hydroxid, -hydrid, -amid, - methanolat, -carbonat, Kalium-tert.-butanolat, -carbonat, Lithiumdiisopropylamid, Kalium-bis-(trimethylsilyl)-amid, Calciumhydrid, Triethylamin, Triethylendiamin, Cyclohexylamin, N-Cyclohexyl-N,N-dimethyl-amin, N,N-Diethylanilin, Pyridin, 4-(N,N-Dimethylamino)-pyridin, N-Methylmorpholin, Benzyl-trimethyl-ammoniumhydroxid sowie 1,5-Diazabicyclo[5.4.0]undec-5-en (DBU) genannt.

Die Umsetzung erfolgt vorteilhaft in einem inerten Lösungs- oder Verdünnungsmittel, beispielsweise der unter der Variante a) angegebenen Art, und in einem Temperaturbereich von etwa -80°C bis etwa +200°C, z.B. von etwa -20°C bis etwa +100°C.

Die Verbindungen der Formel V sind bekannt oder können in Analogie zu bekannten Verbindungen hergestellt werden.

Variante c):

Geeignete saure Katalysatoren sind z.B. von der unter der Variante a) für die Umsetzung von Verbindungen II und III angegebenen Art.

Die Umsetzung erfolgt vorteilhaft in einem inerten Lösungs- oder Verdünnungsmittel, beispielsweise der unter der Variante a) angegebenen Art, und in einem Temperaturbereich von etwa 0°C bis etwa + 180°C, bevorzugt von etwa +20°C bis etwa + 130°C, vielfach bei der Rückflusstemperatur des verwendeten Lösungsmittels.

Die Verbindungen der Formel VI sind bekannt oder können in Analogie zu bekannten Verbindungen hergestellt werden.

Die Verbindungen I können in Form eines der möglichen Isomeren oder als Gemisch derselben, z. B. je nach Anzahl, absoluter und relativer Konfiguration der asymmetrischen Kohlenstoffatome als reine Isomere, wie Antipoden und/oder Diastereomere, oder als Isomerengemische, wie Enantiomerengemische, z. B. Racemate, Diastereomerengemische oder Racematgemische, vorliegen; die Erfindung betrifft sowohl die reinen Isomeren als auch alle möglichen Isomerengemische und ist vor- und nachstehend jeweils entsprechend zu verstehen, auch wenn stereochemische Einzelheiten nicht in jedem Fall speziell erwähnt werden.

Verfahrensgemäss - je nach Wahl der Ausgangsstoffe und Arbeitsweisen - oder anderweitig erhältliche Diastereomerengemische und Racematgemische von Verbindungen I können auf Grund der physikalisch-chemischen Unterschiede der Bestandteile in bekannter Weise in die reinen Diastereomeren oder Racemate aufgetrennt werden, beispielsweise durch fraktionierte Kristallisation, Destillation und/oder Chromatographie.

Entsprechend erhältliche Enantiomerengemische, wie Racemate, lassen sich nach bekannten Methoden in die optischen Antipoden zerlegen, beispielsweise durch Umkristallisation aus einem optisch aktiven Lösungsmittel, durch Chromatographie an chiralen Adsorbentien, z. B. Hochdruckflüssigkeitschromatographie (HPLC) an Acetylcellulose, mit Hilfe von geeigneten Mikroorganismen, durch Spaltung mit spezifischen, immobilisierten Enzymen, über die Bildung von Einschlussverbindungen, z. B. unter Verwendung chiraler Kronenether, wobei nur ein Enantiomeres komplexiert wird.

Ausser durch Auftrennung entsprechender Isomerengemische können reine Diastereomere bzw. Enantiomere erfindungsgemäss auch durch allgemein bekannte Methoden der diastereoselektiven bzw. enantioselektiven Synthese erhalten werden, z. B. indem man das erfindungsgemässe Verfahren mit Edukten mit entsprechend geeigneter Stereochemie ausführt.

Vorteilhaft isoliert bzw. synthetisiert man jeweils das biologisch wirksamere Isomere, z. B. Enantiomere, oder Isomerengemisch, z. B. Enantiomerengemisch, sofern die einzelnen Komponenten unterschiedliche biologische Wirksamkeit besitzen.

Die Verbindungen I können auch in Form ihrer Hydrate erhalten werden und/oder andere, beispielsweise gegebenenfalls zur Kristallisation von in fester Form vorliegenden Verbindungen verwendete, Lösungsmittel einschliessen.

Die Erfindung betrifft alle diejenigen Ausführungsformen des Verfahrens, nach denen man von einer auf irgendeiner Stufe des Verfahrens als Ausgangs- oder Zwischenprodukt erhältlichen Verbindung ausgeht und alle oder einige der fehlenden Schritte durchführt oder einen Ausgangsstoff in Form eines Derivates bzw. Salzes und/oder seiner Racemate bzw. Antipoden verwendet oder insbesondere unter den Reaktionsbedingungen bildet.

Beim Verfahren der vorliegenden Erfindung werden vorzugsweise solche Ausgangsstoffe und Zwischenprodukte verwendet, welche zu den eingangs als besonders wertvoll geschilderten Verbindungen I führen.

Die Erfindung betrifft insbesondere die in den Beispielen H1 bis H5 beschriebenen Herstellungsverfahren.

Erfindungsgemäss für die Herstellung der Verbindungen I verwendete Ausgangsstoffe und Zwischenprodukte, die neu sind, ihre Verwendung und Verfahren zu ihrer Herstellung bilden ebenfalls einen Gegenstand der Erfindung.

In diesem Zusammenhang sind die Verbindungen II von spezieller Bedeutung, welche einen weiteren Gegenstand der vorliegenden Erfindung bilden, ebenso wie ihre Herstellung und ihre Verwendung als Zwischenprodukte.

In 4-Position substituierte Dioxolane ähnlicher Struktur sind bereits bekannt, z. B. aus der europäischen Patentanmeldung mit der Veröffentlichungsnummer 0 276 196 und aus FR-A-2,334,680. Von diesen bekannten Verbindungen unterscheiden sich die Verbindungen I der vorliegenden Erfindung strukturell in charakteristischer Weise dadurch, dass sie in 2-Position des Phenylrings der in der Formel I gezeigten Phenoxymethylgruppe durch $C_1$-$C_3$-Alkyl, Halogen-$C_1$-$C_3$-alkyl, $C_1$-$C_3$-Alkoxy, Halogen-$C_1$-$C_3$-alkoxy, Fluor, Chlor oder Brom ($R_2$) substituiert sind; zudem zeigen die Verbindungen I der vorliegenden Erfindung eine in überraschendem Ausmass gesteigerte Wirksamkeit auf dem Gebiet der Schädlingsbekämpfung.

Die Verbindungen I der vorliegenden Erfindung sind bei Raumtemperatur stabile Oele, Harze oder Feststoffe. Sie sind bei günstiger Warmblüter-, Fisch- und Pflanzenverträglichkeit wertvolle Wirkstoffe auf dem Gebiet der Schädlingsbekämpfung. Insbesondere wirken die erfindungsgemässen Wirkstoffe gegen Insekten und Spinnentiere, wie sie an Nutz- und Zierpflanzen in der Landwirtschaft und im Gartenbau, insbesondere in Reis-, Baumwoll-, Gemüse- und Obstpflanzungen, und im Forst vorkommen. Die Verbindungen I eignen sich besonders zur Bekämpfung von Insekten in Reis-, Obst- und Gemüsekulturen, insbesondere von pflanzenschädigenden Insekten, wie Nilaparvata lugens und Heliothis virescens. Weitere Anwendungsgebiete der erfindungsgemässen Wirkstoffe sind der Vorrats- und Materialschutz sowie im Hygienesektor insbesondere der Schutz von Haus- und Nutztieren. Die Verbindungen I sind gegen alle oder einzelne Entwicklungsstadien von normal sensiblen, aber auch von resistenten Arten von Schädlingen wirksam. Dabei kann sich ihre Wirkung z. B. in einer Abtötung der Schädlinge, welche unmittelbar oder erst nach einiger mit, beispielsweise bei einer Häutung, eintritt, oder in einer verminderten Eiablage und/oder Schlupfrate zeigen.

Zu den oben erwähnten Schädlingen gehören:

aus der Ordnung Lepidoptera zum Beispiel

Acleris spp., Adoxophyes spp., Aegeria spp., Agrotis spp., Alabama argillaceae, Amylois spp., Anticarsia gemmatalis, Archips spp., Argyrotaenia spp., Autographa spp., Busseola fusca, Cadra cautella, Carposina nipponensis, Chilo spp., Choristoneura spp., Clysia ambiguella, Cnaphalocrocis spp., Cnephasia spp., Cochylis spp., Coleophora spp., Crocidolomia binotalis, Cryptophlebia leucotreta, Cydia spp., Diatraea spp., Diparopsis castanea, Earias spp., Ephestia spp., Eucosma spp., Eupoecilia ambiguella, Euproctis spp., Euxoa spp., Grapholita spp., Hedya nubiferana, Heliothis spp., Hellula undalis, Hyphantria cunea, Keiferia lycopersicella, Leucoptera scitella, Lithocollethis spp., Lobesia botrana, Lymantria spp., Lyonetia spp., Malacosoma spp., Mamestra brassicae, Manduca sexta, Operophtera spp., Ostrinia nubilalis, Pammene spp., Pandemis spp., Panolis flammea, Pectinophora gossypiella, Phthorimaea operculella, Pieris rapae, Pieris spp., Plutella xylostella, Prags spp., Scirpophaga spp., Sesamia spp., Sparganothis spp., Spodoptera spp., Synanthedon spp., Thaumetopoea spp., Tortrix spp., Trichoplusia ni und Yponomeuta spp.;

aus der Ordnung Coleoptera zum Beispiel

Agriotes spp., Anthonomus spp., Atomaria linearis, Chaetocnema tibialis, Cosmopolites spp., Curculio spp., Dermestes spp., Diabrotica spp., Epilachna spp., Eremnus spp., Leptinotarsa decemlineata, Lissorhoptrus spp., Melolontha spp., Orycaephilus spp., Otiorhynchus spp., Phlyctinus spp., Popillia spp., Psylliodes spp., Rhizopertha spp., Scarabeidae, Sitophilus spp., Sitotroga spp., Tenebrio spp., Tribolium spp. und Trogoderma spp.;

aus der Ordnung Orthoptera zum Beispiel

Blatta spp., Blattella spp., Gryllotalpa spp., Leucophaea maderae, Locusta spp.,
Periplaneta spp. und Schistocerca spp.;

aus der Ordnung Isoptera zum Beispiel

Reticulitermes spp.;

aus der Ordnung Psocoptera zum Beispiel

Liposcelis spp.;

aus der Ordnung Anoplura zum Beispiel

Haematopinus spp., Linognathus spp., Pediculus spp., Pemphigus spp. und Phylloxera spp.;

aus der Ordnung Mallophaga zum Beispiel

Damalinea spp. und Trichodectes spp.;

aus der Ordnung Thysanoptera zum Beispiel

Frankliniella spp., Hercinothrips spp., Taeniothrips spp., Thrips palmi, Thrips tabaci und
Scirtothrips aurantii;

aus der Ordnung Heteroptera zum Beispiel

Cimex spp., Distantiella theobroma, Dysdercus spp., Euchistus spp., Eurygaster spp., Leptocorisa spp., Nezara spp., Piesma spp., Rhodnius spp., Sahlbergella singularis, Scotinophara spp. und Triatoma spp.;

aus der Ordnung Homoptera zum Beispiel

Aleurothrixus floccosus, Aleyrodes brassicae, Aonidiella spp., Aphididae, Aphis spp., Aspidiotus spp., Bemisia tabaci, Ceroplaster spp., Chrysomphalus aonidium, Chrysomphalus dictyospermi, Coccus hesperidum, Empoasca spp., Eriosoma larigerum, Erythroneura spp., Gascardia spp., Laodelphax spp., Lecanium corni, Lepidosaphes spp., Macrosiphus spp., Myzus spp., Nephotettix spp., Nilaparvata spp., Paratoria spp., Pemphigus spp., Planococcus spp., Pseudaulacaspis spp., Pseudococcus spp., Psylla spp., Pulvinaria aethiopica, Quadraspidiotus spp., Rhopalosiphum spp., Saissetia spp., Scaphoideus spp., Schizaphis spp., Sitobion spp., Trialeurodes vaporariorum, Trioza erytreae und Unaspis citri;

aus der Ordnung Hymenoptera zum Beispiel

Acromyrmex, Atta spp., Cephus spp., Diprion spp., Diprionidae, Gilpinia polytoma, Hoplocampa spp., Lasius spp., Monomorium pharaonis, Neodiprion spp., Solenopsis spp. und Vespa spp.;

aus der Ordnung Diptera zum Beispiel

Aedes spp., Antherigona soccata, Bibio hortulanus, Calliphora erythrocephala, Ceratitis spp., Chrysomyia spp., Culex spp., Cuterebra spp., Dacus spp., Drosophila melanogaster, Fannia spp., Gastrophilus spp., Glossina spp., Hypoderma spp., Hyppobosca spp., Liriomyza spp., Lucilia spp., Melanagromyza spp., Musca spp., Oestrus spp., Orseolia spp., Oscinella frit, Pegomyia hyoscyami, Phorbia spp., Rhagoletis pomonella, Sciara spp., Stomoxys spp., Tabanus spp., Tannia spp. und Tipula spp.;

aus der Ordnung Siphonaptera zum Beispiel

Ceratophyllus spp. und Xenopsylla cheopis;

aus der Ordnung Thysanura zum Beispiel

Lepisma saccharina und

aus der Ordnung Acarina zum Beispiel
Acarus siro, Aceria sheldoni, Aculus schlechtendali, Amblyomma spp., Argas spp., Boophilus spp., Brevipalpus spp., Bryobia praetiosa, Calipitrimerus spp., Chorioptes spp., Dermanyssus gallinae, Eotetranychus carpini, Eriophyes spp., Hyalomma spp., Ixodes spp., Olygonychus pratensis, Ornithodoros spp., Panonychus spp., Phyllocoptruta oleivora, Polyphagotarsonemus latus, Psoroptes spp., Rhipicephalus spp., Rhizoglyphus spp., Sarcoptes spp., Tarsonemus spp. und Tetranychus spp..

Die gute pestizide Wirkung der erfindungsgemässen Verbindungen I entspricht einer Abtötungsrate (Mortalität) von mindestens 50-60 % der erwähnten Schädlinge.

Die Wirkung der erfindungsgemässen Verbindungen I und der sie enthaltenden Mittel lässt sich durch Zusatz von anderen Insektiziden und/oder Akariziden wesentlich verbreitern und an gegebene Umstände anpassen. Als Zusätze kommen zum Beispiel Vertreter der folgenden Wirkstoffklassen in Betracht: Organische Phosphorverbindungen, Nitrophenole und Derivate, Formamidine, Harnstoffe, Carbamate, Pyrethroide, chlorierte Kohlenwasserstoffe und Bacillus thuringiensis-Präparate.

Die Verbindungen I werden in unveränderter Form oder vorzugsweise zusammen mit den in der Formulierungstechnik üblichen Hilfsmitteln eingesetzt und können daher beispielsweise zu emulgierbaren Konzentraten, direkt versprüh- oder verdünnbaren Lösungen, verdünnten Emulsionen, Spritzpulvern, löslichen Pulvern, Stäubemitteln und Granulaten, auch zu Verkapselungen in polymeren Stoffen, in bekannter Weise verarbeitet werden. Die Anwendungsverfalren, wie Versprühen, Vernebeln, Verstäuben, Verstreuen oder Giessen, werden ebenso wie die Mittel den angestrebten Zielen und den gegebenen Verhältnissen entsprechend gewählt. Ferner eignen sich die Verbindungen I auch für den Einsatz bei der Behandlung von Saatgut. Dabei kann sowohl das Saatgut vor dem Säen mit dem Wirkstoff oder einer den Wirkstoff enthaltenden Formulierung behandelt oder gebeizt werden als auch der Wirkstoff beim Säen in die Saatfurche appliziert werden.

Die Formulierungen, das heisst die den Wirkstoff der Formel I, beziehungsweise eine Kombination dieses Wirkstoffs mit anderen Insektiziden und/oder Akariziden, und gegebenenfalls feste oder flüssige Hilfsstoffe enthaltenden Mittel, Zubereitungen oder Zusammensetzungen, werden in bekannter Weise hergestellt, zum Beispiel durch inniges Vermischen und/oder Vermahlen des Wirkstoffs mit den Hilfsstoffen, wie Streckmitteln, z. B. Lösungsmitteln oder festen Trägerstoffen, oder wie oberflächenaktiven Verbindungen (Tensiden).

Als Lösungsmittel können in Frage kommen: Aromatische Kohlenwasserstoffe, bevorzugt die Fraktionen $C_8$ bis $C_{12}$ von Alkylbenzolen, wie Xylolgemische oder alkylierte Naphthaline, aliphatische oder cycloaliphatische Kohlenwasserstoffe, wie Cyclohexan, Paraffine oder Tetrahydronaphthalin, Alkohole wie Aethanol, Propanol oder Butanol, Glykole sowie deren Aether und Ester, wie Propylenglykol, Dipropylenglykoläther, Aethylenglykol, Aethylenglykolmonomethyl- oder -äthyläther, Ketone, wie Cyclohexanon, Isophoron oder Diacetonalkohol, stark polare Lösungsmittel, wie N-Methyl-2-pyrrolidon, Dimethylsulfoxid oder N,N-Dimethylformamid, Wasser sowie gegebenenfalls epoxidierte Pflanzenöle, wie gegebenenfalls epoxidiertes Raps-, Rizinus-, Kokosnuss- oder Sojaöl; gegebenenfalls auch Silikonöle.

Als feste Trägerstoffe, beispielsweise für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden. Als gekörnte, adsorptive Granulatträger kommen poröse Typen, wie Bimsstein, Ziegelbruch, Sepiolit oder Bentonit, als nicht sorptive Trägermaterialien Calcit oder Sand in Frage. Darüberhinaus kann eine Vielzahl von granulierten Materialien anorganischer oder organischer Natur, insbesondere Dolomit oder zerkleinerte Pflanzenrückstände, verwendet werden.

Als oberflächenaktive Verbindungen kommen je nach Art des zu formulierenden Wirkstoffs I oder der Kombination dieses Wirkstoffs mit anderen Insektiziden und/oder Akariziden nichtionische, kationische und/oder anionische Tenside mit guten Emulgier-, Dispergier- und Netzeigenschaften in Betracht. Unter Tensiden sind auch Tensidgemische zu verstehen.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignet sind die wasserlöslichen 20 bis 250 Aethylenglykoläthergruppen und 10 bis 100 Propylenglykoläthergruppen enthaltenden Polyäthylenoxid-Addukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykol-Einheiten. Als Beispiele nichtionischer Tenside seien Nonylphenolpolyäthoxyäthanole, Ricinusölpolyglykoläther, Polypropylen-Polyäthylenoxid-Addukte, Tributylphenoxypolyäthoxyäthanol, Polyäthylenglykol und Octylphenoxypolyäthoxyäthanol erwähnt. Ferner kommen auch Fettsäureester von Polyoxyäthylensorbitan, wie das Polyoxyäthylensorbitan-trioleat, in Betracht.

Bei den kationischen Tensiden handelt es sich vor allem um quarternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als

Halogenide, Methylsulfate oder Aethylsulfate vor, Beispiele sind das Stearyltrimethylammoniumchlorid oder das Benzyl-di-(2-chloräthyl)-äthylammoniumbromid.

Geeignete anionische Tenside können sowohl sogenannte wasserlösliche Seifen als auch wasserlösliche synthetische oberflächenaktive Verbindungen sein. Als Seifen eignen sich die Alkali-, Erdalkali- oder gegebenenfalls substituierten Ammoniumsalze von höheren Fettsäuren ($C_{10}$-$C_{22}$), wie die Natrium- oder Kalium-Salze der Oel- oder Stearinsäure oder von natürlichen Fettsäuregemischen, die beispielsweise aus Kokosnuss- oder Tallöl gewonnen werden können; ferner sind auch die Fettsäuremethyl-taurin-salze zu erwähnen. Häufiger werden jedoch sogenannte synthetische Tenside verwendet, insbesondere Fettsulfonate, Fettsulfate, sulfonierte Benzimidazolderivate oder Alkylarylsulfonate. Die Fettsulfonate oder -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst; beispielhaft genannt seien das Natrium- oder Calcium-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit etwa 8-22 C-Atomen. Alkylarylsulfonate sind zum Beispiel die Natrium-, Calcium- oder Triäthanolammoniumsalze salze der Dodecylbenzolsulfonsäure, der Dibutylnaphthalinsulfonsäure oder eines Naphthalinsulfonsäure-Formaldehydkondensationsproduktes. Ferner kommen auch entsprechende Phosphate, wie Salze des Phosphorsäureesters eines p-Nonylphenol-(4-14)-Aethylenoxid-Adduktes oder Phospholipide, in Frage.

Die vorstehend aufgeführten Tenside sind nur als Beispiele anzusehen; in der einschlägigen Literatur werden viele weitere in der Formulierungstechnik gebräuchliche und erfindungsgemäss geeignete Tenside beschrieben.

Die pestiziden Zubereitungen enthalten in der Regel 0,1 bis 99%, insbesondere 0,1 bis 95%, an Wirkstoff I oder an der Kombination dieses Wirkstoffs mit anderen Insektiziden und/oder Akariziden und 1 bis 99,9%, insbesondere 5 bis 99,9%, eines festen oder flüssigen Hilfsstoffes, wobei in der Regel 0 bis 25%, insbesondere 0,1 bis 20%, der Zubereitungen Tenside sein können (% bedeutetjeweils Gewichtsprozent). Während als Handelsware eher konzentrierte Mittel bevorzugt werden, verwendet der Endverbraucher in der Regel verdünnte Zubereitungen, die wesentlich geringere Wirkstoffkonzentrationen aufweisen. Typische Anwendungskonzentrationen liegen zwischen 0,1 und 1000 ppm, vorzugsweise zwischen 0,1 und 500 ppm. Die Aufwandmengen pro Hektar betragen im allgemeinen 1 bis 1000 g Wirkstoff pro Hektar, vorzugsweise 25 bis 500 g/ha.

Insbesondere setzen sich bevorzugte Formulierungen folgendermassen zusammen (% = Gewichtsprozent):

Emulgierbare Konzentrate:

| | |
|---|---|
| Wirkstoff: | 1 bis 90 %, bevorzugt 5 bis 20 % |
| Tensid: | 1 bis 30 %, vorzugsweise 10 bis 20 % |
| flüssiger Trägerstoff: | 5 bis 94 %, vorzugsweise 70 bis 85 % |

Stäube:

| | |
|---|---|
| Wirkstoff: | 0,1 bis 10 %, vorzugsweise 0,1 bis 1 % |
| fester Trägerstoff: | 99,9 bis 90 %, vorzugsweise 99,9 bis 99 % |

Suspensions-Konzentrate:

| | |
|---|---|
| Wirkstoff: | 5 bis 75 %, vorzugsweise 10 bis 50 % |
| Wasser: | 94 bis 24 %, vorzugsweise 88 bis 30 % |
| Tensid: | 1 bis 40 %, vorzugsweise 2 bis 30 % |

Benetzbare Pulver:

| | |
|---|---|
| Wirkstoff: | 0,5 bis 90 %, vorzugsweise 1 bis 80 % |
| Tensid: | 0,5 bis 20 %, vorzugsweise 1 bis 15 % |
| fester Trägerstoff: | 5 bis 95 %, vorzugsweise 15 bis 90 % |

Granulate:

| | |
|---|---|
| Wirkstoff: | 0,5 bis 30 %, vorzugsweise 3 bis 15 % |
| fester Trägerstoff: | 99,5 bis 70 %, vorzugsweise 97 bis 85 % |

Die Zubereitungen können auch weitere Hilfsstoffe, wie Stabilisatoren, z. B. gegebenenfalls epoxidierte Pflanzenöle (z. B. epoxidiertes Kokosnussöl, Rapsöl oder Sojaöl), Entschäumer, z. B. Silikonöl, Konservierungsmittel, Viskositätsregulatoren, Bindemittel und/oder Haftmittel, sowie Dünger oder andere Wirkstoffe zur Erzielung spezieller Effekte enthalten.

Eingeschlossen in die Erfindung ist auch ein Verfahren zur Bekämpfung der vorstehend erwähnten Schädlinge, das sich durch Applikation eines Wirkstoffs der Formel I oder eines erfindungsgemässen Mittels, enthaltend einen Wirkstoff der Formel I, auf die Schädlinge bzw. deren verschiedene Entwicklungsstadien oder ihren Aufenthaltsort auszeichnet.

Die folgenden Beispiele illustrieren die vorstehend beschriebene Erfindung, ohne dieselbe in ihrem Umfang in irgendeiner Weise einzuschränken. " $n_D^T$ " steht für den Brechungsindex bei einer Temperatur von T°C. Temperaturen sind in Celsiusgraden angegeben. "Smp." steht für Schmelzpunkt; "%" bedeutet Gewichtsprozent, sofern nichts anderes angegeben ist. Verbindungen I, worin $R_1$ und $R_4$ verschieden sind, können Diastereomere bilden; "A" und "B" stehen jeweils für die beiden aufgetrennten Diastereomeren-Komponenten solcher Gemische; fehlen hingegen die Angaben "A" und "B", so handelt es sich bei dem Produkt um ein Gemisch der Diastereomeren.

Herstellungsbeispiele

Beispiel H1: 3-(2-Chlor-4-phenoxy-phenoxy)-1,2-dihydroxy-propan

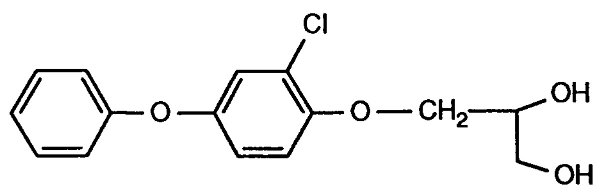

Zu einer Lösung von 22,1 g (0,1 mol) 2-Chlor-4-phenoxy-phenol in 20 ml Xylol gibt man 0,2 g Tetramethylammoniumchlorid, erwärmt das Gemisch auf 60°C und lässt unter Rühren innert 20 Minuten 8,1 g 2,3-Epoxypropan-1-ol zutropfen. Danach wird das Reaktionsgemisch 8 Stunden bei 90°C nachgerührt, bei 60°C innert 5 Minuten unter Rühren mit 60 ml Hexan versetzt und auf 0°C abgekühlt und der ausgefallene Niederschlag abfiltriert. Das erhaltene Rohprodukt wird in Isopropanol/Hexan umkristallisiert. Man erhält farblose Kristalle der Titelverbindung mit einem Schmelzpunkt von 87-88°C (Verbindung Nr. 1.13).

Beispiel H2: In analoger Weise wie in Beispiel H1 angegeben, gegebenenfalls nach zusätzlicher chromatographischer Reinigung [$CH_2Cl_2/(C_2H_5)_2O$ = 3:2], kann man auch die weiteren in der Tabelle 1 aufgeführten Verbindungen der Formel II herstellen.

Tabelle 1

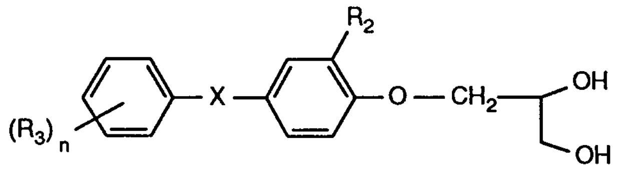

| Verb. Nr. | $R_2$ | $(R_3)_n$ | X | phys. Daten |
|---|---|---|---|---|
| 1.1 | Cl | 3-F | O | $n_D^{20} = 1.5782$ |
| 1.2 | Cl | 3-Cl | O | $n_D^{20} = 1.5940$ |
| 1.3 | Cl | 3-F,5-F | O | Smp. = 52-53° |
| 1.4 | Cl | 3-Cl,4-F | O | $n_D^{20} = 1.5706$ |
| 1.5 | Cl | 3-Cl,4-Cl | O | Smp. = 61-62° |
| 1.6 | Cl | 3-Cl,5-Cl | O | |
| 1.7 | Cl | 4-F | O | $n_D^{20} = 1.5762$ |
| 1.8 | Cl | 4-Cl | O | $n_D^{20} = 1.5920$ |
| 1.9 | Cl | $3-CH_3$ | O | $n_D^{20} = 1.5671$ |
| 1.10 | Cl | 2-F,4-F | O | |
| 1.11 | $CH_3$ | n = 0 | O | $n_D^{20} = 1.5724$ |
| 1.12 | $CH_3$ | 3-F | O | $n_D^{20} = 1.5621$ |
| 1.13 | Cl | n = 0 | O | Smp. = 87-88° |
| 1.14 | $CH_3$ | 3-F,5-F | O | $n_D^{20} = 1.5702$ |
| 1.15 | $CH_3$ | 3-Cl | O | $n_D^{20} = 1.5794$ |
| 1.16 | $CH_3$ | 3-Cl,4-Cl | O | Smp. = 42-43° |
| 1.17 | $CH_3$ | 3-Cl,4-F | O | |
| 1.18 | Cl | n = 0 | $CH_2$ | Smp. = 75-77° |
| 1.19 | $CH_3$ | n = 0 | $CH_2$ | Smp. = 73-74° |
| 1.20 | Cl | 3-F | $CH_2$ | Smp. = 80-81° |
| 1.21 | $CH_3$ | 3-F | $CH_3$ | $n_D^{20} = 1.5621$ |
| 1.22 | Cl | 3-F,5-F | $CH_2$ | Smp. = 89-90° |
| 1.23 | Cl | 3-Cl,4-Cl | $CH_2$ | Smp. = 97-98° |
| 1.24 | Br | n = 0 | O | Smp. = 79-80° |
| 1.25 | Br | 3-F | O | $n_D^{20} = 1.5869$ |
| 1.26 | Br | n = 0 | $CH_2$ | Smp. = 89-90° |
| 1.27 | Cl | n = 0 | C=O | $n_D^{20} = 1.6139$ |
| 1.28 | Cl | 4-Cl | C=O | Smp. = 112-113° |
| 1.29 | F | n = 0 | O | Smp. = 74-75° |

| Verb. Nr. | $R_2$ | $(R_3)_n$ | X | phys. Daten |
|---|---|---|---|---|
| 1.30 | F | 3-Cl | O | $n_D^{20} = 1.5671$ |
| 1.31 | F | 3-F | O | $n_D^{20} = 1.5529$ |
| 1.32 | Br | 3-Cl,4-Cl | $CH_2$ | |
| 1.33 | F | 3-F | $CH_2$ | |

Beispiel H3: 4-(2-Chlor-4-phenoxy-phenoxymethyl)-2-ethyl-1,3-dioxolan

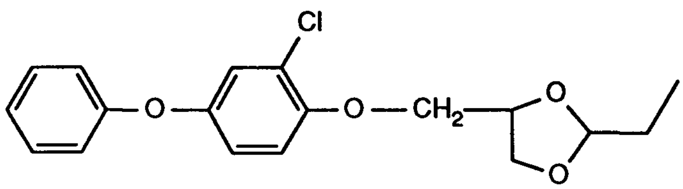

Zu einer Lösung von 5.9 g (0,02 mol) 3-(2-Chlor-4-phenoxy-phenoxy)- 1,2-dihydroxypropan und 20 mg 4-Toluolsulfonsäure in 40 ml Toluol lässt man unter Rühren bei Rückflusstemperatur 1,6 g (0,028 mol) frisch destilliertes Propanal zutropfen. Das Gemisch wird 6 Stunden bei Rückflusstemperatur nachgerührt und dann wiederholt mit 10%iger Natriumcarbonatlösung und anschliessend mit Wasser gewaschen. Die Toluolphase wird über Natriumsulfat getrocknet und im Vakuum vom Lösungsmittel befreit. Durch chromatographische Reinigung des Rohproduktes an Kieselgel [Hexan:Diethylether (15:1)], erhält man die Titelverbindung in Form von zwei getrennten Diastereomeren [Diastereomer A (Verbindung Nr. 2.3.1): $n_D^{20}$ = 1.5591; Diastereomer B (Verbindung Nr. 2.3.2): $n_D^{20}$ = 1.5611].

Beispiel H4: In analoger Weise wie in Beispiel H3 beschrieben kann man auch die anderen in den Tabellen 2 bis 8 aufgeführten Verbindungen der Formel I herstellen.

Tabelle 2

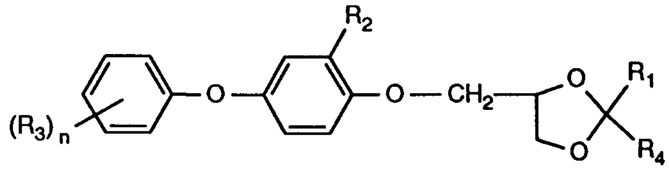

| Verb. Nr. | $R_1$ | $R_2$ | $(R_3)_n$ | $R_4$ | phys. Daten |
|---|---|---|---|---|---|
| 2.1 | $CH_3$ | Cl | n = 0 | $CH_3$ | $n_D^{20}$ = 1.5564 |
| 2.2 | $CH_3$ | Cl | n = 0 | H | |
| 2.3 | $C_2H_5$ | Cl | n = 0 | H | |
| 2.3.1 | $C_2H_5$ | Cl | n = 0 | H | A; $n_D^{20}$ = 1.5591 |
| 2.3.2 | $C_2H_5$ | Cl | n = 0 | H | B; $n_D^{20}$ = 1.5611 |
| 2.4 | $C_3H_7$ | Cl | n = 0 | H | |
| 2.4.1 | $C_3H_7$ | Cl | n = 0 | H | A; $n_D^{20}$ = 1.5539 |
| 2.4.2 | $C_3H_7$ | Cl | n = 0 | H | B; $n_D^{20}$ = 1.5552 |
| 2.5 | i-$C_3H_7$ | Cl | n = 0 | H | $n_D^{20}$ = 1.5524 |
| 2.6 | i-$C_4H_9$ | Cl | n = 0 | H | |
| 2.7 | s-$C_4H_9$ | Cl | n = 0 | H | |
| 2.8 | cyclo-$C_3H_5$ | Cl | n = 0 | H | |
| 2.8.1 | cyclo-$C_3H_5$ | Cl | n = 0 | H | A; $n_D^{20}$ = 1.5693 |
| 2.8.2 | cyclo-$C_3H_5$ | Cl | n = 0 | H | B; $n_D^{20}$ = 1.5702 |
| 2.9 | $C_2H_5$ | $CH_3$ | n = 0 | H | |
| 2.9.1 | $C_2H_5$ | $CH_3$ | n = 0 | H | A; $n_D^{20}$ = 1.5463 |
| 2.9.2 | $C_2H_5$ | $CH_3$ | n = 0 | H | B; $n_D^{20}$ = 1.5481 |
| 2.10 | $C_3H_7$ | $CH_3$ | n = 0 | H | |
| 2.10.1 | $C_3H_7$ | $CH_3$ | n = 0 | H | A; $n_D^{20}$ = 1.5408 |
| 2.10.2 | $C_3H_7$ | $CH_3$ | n = 0 | H | B; $n_D^{20}$ = 1.5421 |
| 2.11 | i-$C_3H_7$ | $CH_3$ | n = 0 | H | |
| 2.12 | cyclo-$C_3H_5$ | $CH_3$ | n = 0 | $CH_3$ | |
| 2.13 | $CH_3$ | Cl | 3-F | $CH_3$ | |
| 2.14 | $CH_3$ | Cl | 3-F | H | |
| 2.14.1 | $CH_3$ | Cl | 3-F | H | A; $n_D^{20}$ = 1.5529 |
| 2.14.2 | $CH_3$ | Cl | 3-F | H | B; $n_D^{20}$ = 1.5541 |
| 2.15 | $C_2H_5$ | Cl | 3-F | H | |
| 2.15.1 | $C_2H_5$ | Cl | 3-F | H | A; $n_D^{20}$ = 1.5481 |

| Verb. Nr. | $R_1$ | $R_2$ | $(R_3)_n$ | $R_4$ | phys. Daten |
|---|---|---|---|---|---|
| 2.15.2 | $C_2H_5$ | Cl | 3-F | H | B; $n_D^{20}$ = 1.5501 |
| 2.16 | $C_3H_7$ | Cl | 3-F | H | |
| 2.16.1 | $C_3H_7$ | Cl | 3-F | H | A; $n_D^{20}$ = 1.5432 |
| 2.16.2 | $C_3H_7$ | Cl | 3-F | H | B; $n_D^{20}$ = 1.5456 |
| 2.17 | i-$C_3H_7$ | Cl | 3-F | H | |
| 2.17.1 | i-$C_3H_7$ | Cl | 3-F | H | A; $n_D^{20}$ = 1.5422 |
| 2.17.2 | i-$C_3H_7$ | Cl | 3-F | H | B; $n_D^{20}$ = 1.5431 |
| 2.18 | s-$C_4H_9$ | Cl | 3-F | H | |
| 2.19 | i-$C_4H_9$ | Cl | 3-F | H | |
| 2.19.1 | i-$C_4H_9$ | Cl | 3-F | H | A; $n_D^{20}$ = 1.5382 |
| 2.19.2 | i-$C_4H_9$ | Cl | 3-F | H | B; $n_D^{20}$ = 1.5392 |
| 2.20 | cyclo-$C_3H_5$ | Cl | 3-F | H | |
| 2.20.1 | cyclo-$C_3H_5$ | Cl | 3-F | H | A; $n_D^{20}$ = 1.5592 |
| 2.20.2 | cyclo-$C_3H_5$ | Cl | 3-F | H | B; $n_D^{20}$ = 1.5612 |
| 2.21 | $C_2H_5$ | $CH_3$ | 3-F | H | $n_D^{20}$ = 1.5371 |
| 2.22 | $C_3H_7$ | $CH_3$ | 3-F | H | $n_D^{20}$ = 1.5332 |
| 2.23 | i-$C_3H_7$ | $CH_3$ | 3-F | H | |
| 2.24 | $CH_3$ | Cl | 3-F,5-F | $CH_3$ | |
| 2.25 | $CH_3$ | Cl | 3-F,5-F | H | |
| 2.26 | $C_2H_5$ | Cl | 3-F,5-F | H | |
| 2.26.1 | $C_2H_5$ | Cl | 3-F,5-F | H | A; $n_D^{20}$ = 1.5369 |
| 2.26.2 | $C_2H_5$ | Cl | 3-F,5-F | H | B; $n_D^{20}$ = 1.5379 |
| 2.27 | $C_3H_7$ | Cl | 3-F,5-F | H | |
| 2.27.1 | $C_3H_7$ | Cl | 3-F,5-F | H | A; $n_D^{20}$ = 1.5330 |
| 2.27.2 | $C_3H_7$ | Cl | 3-F,5-F | H | B; $n_D^{20}$ = 1.5339 |
| 2.28 | i-$C_3H_7$ | Cl | 3-F,5-F | H | |
| 2.28.1 | i-$C_3H_7$ | Cl | 3-F,5-F | H | A; $n_D^{20}$ = 1.5325 |
| 2.28.2 | i-$C_3H_7$ | Cl | 3-F,5-F | H | B; $n_D^{20}$ = 1.5331 |
| 2.29 | i-$C_4H_9$ | Cl | 3-F,5-F | H | |
| 2.30 | $C_4H_9$ | Cl | 3-F,5-F | H | |
| 2.31 | s-$C_4H_9$ | Cl | 3-F,5-F | H | |
| 2.32 | cyclo-$C_3H_5$ | Cl | 3-F,5-F | H | |
| 2.32.1 | cyclo-$C_3H_5$ | Cl | 3-F,5-F | H | A; $n_D^{20}$ = 1.5461 |
| 2.32.2 | cyclo-$C_3H_5$ | Cl | 3-F,5-F | H | B; $n_D^{20}$ = 1.5470 |

| Verb. Nr. | $R_1$ | $R_2$ | $(R_3)_n$ | $R_4$ | phys. Daten |
|---|---|---|---|---|---|
| 2.33 | $C_2H_5$ | $CH_3$ | 3-F,5-F | H | |
| 2.34 | $C_3H_7$ | $CH_3$ | 3-F,5-F | H | |
| 2.35 | i-$C_3H_7$ | $CH_3$ | 3-F,5-F | H | |
| 2.36 | cyclo-$C_3H_5$ | $CH_3$ | 3-F,5-F | $CH_3$ | |
| 2.37 | $CH_3$ | Cl | 3-Cl | $CH_3$ | |
| 2.38 | $C_2H_5$ | Cl | 3-Cl | H | |
| 2.38.1 | $C_2H_5$ | Cl | 3-Cl | H | A; $n_D^{20}$ = 1.5662 |
| 2.38.2 | $C_2H_5$ | Cl | 3-Cl | H | B; $n_D^{20}$ = 1.5679 |
| 2.39 | $C_3H_7$ | Cl | 3-Cl | H | |
| 2.39.1 | $C_3H_7$ | Cl | 3-Cl | H | A; $n_D^{20}$ = 1.5604 |
| 2.39.2 | $C_3H_7$ | Cl | 3-Cl | H | B; $n_D^{20}$ = 1.5624 |
| 2.40 | i-$C_3H_7$ | Cl | 3-Cl | H | |
| 2.41 | cyclo-$C_3H_5$ | Cl | 3-Cl | H | |
| 2.42 | $C_2H_5$ | $CH_3$ | 3-Cl | H | $n_D^{20}$ = 1.5561 |
| 2.43 | $CH_3$ | Cl | 4-F | $CH_3$ | |
| 2.44 | $C_2H_5$ | Cl | 4-F | H | |
| 2.45 | $C_3H_7$ | Cl | 4-F | H | |
| 2.46 | i-$C_3H_7$ | Cl | 4-F | H | |
| 2.47 | cyclo-$C_3H_5$ | Cl | 4-F | H | |
| 2.48 | $CH_3$ | Cl | 4-Cl | H | |
| 2.49 | $C_2H_5$ | Cl | 4-Cl | H | |
| 2.50 | $C_3H_7$ | Cl | 4-Cl | H | |
| 2.51 | i-$C_3H_7$ | Cl | 4-Cl | H | |
| 2.52 | cyclo-$C_3H_5$ | Cl | 4-Cl | H | |
| 2.53 | $CH_3$ | Cl | 3-$CH_3$ | $CH_3$ | |
| 2.54 | $CH_3$ | Cl | 3-$CH_3$ | H | |
| 2.55 | $C_2H_5$ | Cl | 3-$CH_3$ | H | |
| 2.56 | $C_3H_7$ | Cl | 3-$CH_3$ | H | |
| 2.57 | i-$C_3H_7$ | Cl | 3-$CH_3$ | H | |
| 2.58 | cyclo-$C_3H_5$ | Cl | 3-$CH_3$ | H | |
| 2.59 | $CH_3$ | Cl | 3-Cl,4-Cl | $CH_3$ | |
| 2.60 | $CH_3$ | Cl | 3-Cl,4-Cl | H | |
| 2.61 | $C_2H_5$ | Cl | 3-Cl,4-Cl | H | |
| 2.61.1 | $C_2H_5$ | Cl | 3-Cl,4-Cl | H | A; $n_D^{20}$ = 1.5749 |

| Verb. Nr. | $R_1$ | $R_2$ | $(R_3)_n$ | $R_4$ | phys. Daten |
|---|---|---|---|---|---|
| 2.61.2 | $C_2H_5$ | Cl | 3-Cl,4-Cl | H | B; $n_D^{20}$ = 1.5758 |
| 2.62 | $C_3H_7$ | Cl | 3-Cl,4-Cl | H | |
| 2.62.1 | $C_3H_7$ | Cl | 3-Cl,4-Cl | H | A; $n_D^{20}$ = 1.5691 |
| 2.62.2 | $C_3H_7$ | Cl | 3-Cl,4-Cl | H | B; $n_D^{20}$ = 1.5705 |
| 2.63 | i-$C_3H_7$ | Cl | 3-Cl,4-Cl | H | |
| 2.64 | i-$C_4H_9$ | Cl | 3-Cl,4-Cl | H | |
| 2.65 | cyclo-$C_3H_5$ | Cl | 3-Cl,4-Cl | H | |
| 2.65.1 | cyclo-$C_3H_5$ | Cl | 3-Cl,4-Cl | H | A; $n_D^{20}$ = 1.5798 |
| 2.65.2 | cyclo-$C_3H_5$ | Cl | 3-Cl,4-Cl | H | B; $n_D^{20}$ = 1.5815 |
| 2.66 | cyclo-$C_3H_5$ | Cl | 3-Cl,4-Cl | $CH_3$ | |
| 2.67 | $C_2H_5$ | $CH_3$ | 3-Cl,4-Cl | H | |
| 2.68 | $C_3H_7$ | $CH_3$ | 3-Cl,4-Cl | H | |
| 2.69 | $CH_3$ | Cl | 3-Cl,4-F | $CH_3$ | |
| 2.70 | $CH_3$ | Cl | 3-Cl,4-F | H | |
| 2.71 | $C_2H_5$ | Cl | 3-Cl,4-F | H | |
| 2.71.1 | $C_2H_5$ | Cl | 3-Cl,4-F | H | A; $n_D^{20}$ = 1.5561 |
| 2.71.2 | $C_2H_5$ | Cl | 3-Cl,4-F | H | B; $n_D^{20}$ = 1.5572 |
| 2.72 | $C_3H_7$ | Cl | 3-Cl,4-F | H | |
| 2.73 | i-$C_3H_7$ | Cl | 3-Cl,4-F | H | |
| 2.74 | cyclo-$C_3H_5$ | Cl | 3-Cl,4-F | H | |
| 2.75 | cyclo-$C_3H_5$ | Cl | 3-Cl,4-F | $CH_3$ | |
| 2.76 | $C_2H_5$ | $CH_3$ | 3-Cl,4-F | H | |
| 2.77 | $CH_3$ | Cl | 2-F,4-F | $CH_3$ | |
| 2.78 | $CH_3$ | Cl | 2-F,4-F | H | |
| 2.79 | $C_2H_5$ | Cl | 2-F,4-F | H | |
| 2.80 | $C_3H_7$ | Cl | 2-F,4-F | H | |
| 2.81 | i-$C_3H_7$ | Cl | 2-F,4-F | H | |
| 2.82 | cyclo-$C_3H_5$ | Cl | 2-F,4-F | H | |
| 2.83 | i-$C_4H_9$ | Cl | 3-Cl | H | |
| 2.83.1 | i-$C_4H_9$ | Cl | 3-Cl | H | A; $n_D^{20}$ = 1.5541 |
| 2.83.2 | i-$C_4H_9$ | Cl | 3-Cl | H | B; $n_D^{20}$ = 1.5552 |
| 2.84 | $C_2H_5$ | Br | H | H | |
| 2.84.1 | $C_2H_5$ | Br | H | H | A; $n_D^{20}$ = 1.5738 |
| 2.84.2 | $C_2H_5$ | Br | H | H | B; $n_D^{20}$ = 1.5754 |

| Verb. Nr. | $R_1$ | $R_2$ | $(R_3)_n$ | $R_4$ | phys. Daten |
|---|---|---|---|---|---|
| 2.85 | $R_1$+$R_4$: $-(CH_2)_3-$ | Cl | 3-F | | $n_D^{20}$ = 1.5586 |
| 2.86 | $C_2H_5$ | Br | 3-F | H | |
| 2.86.1 | $C_2H_5$ | Br | 3-F | H | A; $n_D^{20}$ = 1.5584 |
| 2.86.2 | $C_2H_5$ | Br | 3-F | H | B; $n_D^{20}$ = 1.5611 |
| 2.87 | $C_3H_7$ | Br | 3-F | H | |
| 2.87.1 | $C_3H_7$ | Br | 3-F | H | A; $n_D^{20}$ = 1.5529 |
| 2.87.2 | $C_3H_7$ | Br | 3-F | H | B; $n_D^{20}$ = 1.5561 |
| 2.88 | $CH_3$ | Cl | 3-Cl | H | |
| 2.88.1 | $CH_3$ | Cl | 3-Cl | H | A; $n_D^{20}$ = 1.5701 |
| 2.88.2 | $CH_3$ | Cl | 3-Cl | H | B; $n_D^{20}$ = 1.5705 |
| 2.89 | $R_1$+$R_4$: $-(CH_2)_5-$ | Cl | 3-Cl | | $n_D^{20}$ = 1.5631 |
| 2.90 | $R_1$+$R_4$: $-(CH_2)_4-$ | Cl | 3-Cl | | $n_D^{20}$ = 1.5721 |
| 2.91 | $R_1$+$R_4$: $-(CH_2)_4-$ | Cl | 3-F | | $n_D^{20}$ = 1.5539 |
| 2.92 | $R_1$+$R_4$: $-(CH_2)_5-$ | Cl | 3-F | | $n_D^{20}$ = 1.5558 |
| 2.93 | $OCH_3$ | Cl | H | H | |
| 2.93.1 | $OCH_3$ | Cl | H | H | A; $n_D^{20}$ = 1.5646 |
| 2.93.2 | $OCH_3$ | Cl | H | H | B; $n_D^{20}$ = 1.5650 |
| 2.94 | $OCH_3$ | Cl | 3-Cl | H | |
| 2.94.1 | $OCH_3$ | Cl | 3-Cl | H | A; $n_D^{20}$ = 1.5700 |
| 2.94.2 | $OCH_3$ | Cl | 3-Cl | H | B; $n_D^{20}$ = 1.5706 |
| 2.95 | $OCH_3$ | Cl | 3-F | H | |
| 2.95.1 | $OCH_3$ | Cl | 3-F | H | A; $n_D^{20}$ = 1.5530 |
| 2.95.2 | $OCH_3$ | Cl | 3-F | H | B; $n_D^{20}$ = 1.5536 |
| 2.96 | $-CH=CH_2$ | Cl | 3-F | H | |
| 2.96.1 | $-CH=CH_2$ | Cl | 3-F | H | A; $n_D^{20}$ = 1.5572 |
| 2.96.2 | $-CH=CH_2$ | Cl | 3-F | H | B; $n_D^{20}$ = 1.5579 |
| 2.97 | $-CH=CH_2$ | Cl | H | H | |
| 2.97.1 | $-CH=CH_2$ | Cl | H | H | A; $n_D^{20}$ = 1.5681 |
| 2.97.2 | $-CH=CH_2$ | Cl | H | H | B; $n_D^{20}$ = 1.5698 |
| 2.98 | Ethinyl | Cl | 3-Cl | H | $n_D^{20}$ = 1.5798 |
| 2.99 | $C_3H_7$ | $CH_3$ | 3-Cl | H | $n_D^{20}$ = 1.5511 |
| 2.100 | $C_3H_7$ | F | H | H | |
| 2.100.1 | $C_3H_7$ | F | H | H | A; $n_D^{20}$ = 1.5355 |
| 2.100.2 | $C_3H_7$ | F | H | H | B; $n_D^{20}$ = 1.5369 |

| Verb. Nr. | $R_1$ | $R_2$ | $(R_3)_n$ | $R_4$ | phys. Daten |
|---|---|---|---|---|---|
| 2.101 | $C_2H_5$ | F | 3-Cl | H | |
| 2.101.1 | $C_2H_5$ | F | 3-Cl | H | A; $n_D^{20} = 1.5481$ |
| 2.101.2 | $C_2H_5$ | F | 3-Cl | H | B; $n_D^{20} = 1.5491$ |
| 2.102 | $C_3H_7$ | F | 3-Cl | H | |
| 2.102.1 | $C_3H_7$ | F | 3-Cl | H | A; $n_D^{20} = 1.5431$ |
| 2.102.2 | $C_3H_7$ | F | 3-Cl | H | B; $n_D^{20} = 1.5448$ |
| 2.103 | $C_2H_5$ | F | 3-F | H | |
| 2.103.1 | $C_2H_5$ | F | 3-F | H | A; $n_D^{20} = 1.5291$ |
| 2.103.2 | $C_2H_5$ | F | 3-F | H | B; $n_D^{20} = 1.5306$ |
| 2.104 | $C_3H_7$ | F | 3-F | H | |
| 2.104.1 | $C_3H_7$ | F | 3-F | H | A; $n_D^{20} = 1.5248$ |
| 2.104.2 | $C_3H_7$ | F | 3-F | H | B; $n_D^{20} = 1.5262$ |
| 2.105 | $C_6H_{13}$ | Cl | n = 0 | H | |
| 2.106 | $C_5H_{11}$ | Cl | 3-Cl | H | |
| 2.107 | s-$C_4H_9$ | Cl | 3-Cl | H | |
| 2.108 | $C_2H_5$ | Cl | 3-F | $CH_3$ | |
| 2.109 | cyclo-$C_3H_5$ | Cl | 3-Cl | $CH_3$ | |
| 2.110 | $C_2H_5$ | Cl | 3-Cl | $CH_3$ | |
| 2.111 | $-CH_2CH_2F$ | Cl | n = 0 | H | |
| 2.112 | $-CH_2CH_2F$ | Cl | 3-Cl | H | |
| 2.113 | $-CH_2CH_2F$ | Cl | 3-F | H | |
| 2.114 | $-CH_2CH_2F$ | Cl | 3-F,5-F | H | |
| 2.115 | $-CH_2CH_2F$ | F | 3-F | H | |
| 2.116 | $-CH_2CH_2F$ | $CH_3$ | 3-Cl | H | |
| 2.117 | $-CH_2CH_2F$ | F | 3-Cl | H | |
| 2.118 | $-CH_2CH_2F$ | F | n = 0 | H | |
| 2.119 | $-CH_2CH_2Cl$ | Cl | n = 0 | H | |
| 2.120 | $-CH_2CH_2Cl$ | Cl | 3-F | H | |
| 2.121 | $-CH_2CH_2Cl$ | Cl | 3-Cl | H | |
| 2.122 | $-CH=CH_2$ | Cl | 3-Cl | H | |
| 2.123 | $-CH=CH_2$ | Cl | 3-F,5-F | H | |
| 2.124 | $-CH=CHCH_3$ (E) | Cl | 3-Cl | H | |
| 2.125 | $-CH=CHCH_3$ (Z) | Cl | 3-Cl | H | |
| 2.126 | $-CH=CHCH_3$ (E/Z) | F | 3-Cl | H | |

| Verb. Nr. | $R_1$ | $R_2$ | $(R_3)_n$ | $R_4$ | phys. Daten |
|---|---|---|---|---|---|
| 2.127 | $-CH{=}C(CH_3)_2$ | Cl | n = 0 | H | |
| 2.128 | $-CH{=}C(CH_3)_2$ | Cl | 3-Cl | H | |
| 2.129 | $-CH{=}C(CH_3)_2$ | Cl | 3-F | H | |
| 2.130 | $i-C_4H_9$ | F | n = 0 | H | |
| 2.131 | $i-C_4H_9$ | F | 3-Cl | H | |
| 2.132 | $i-C_4H_9$ | $CH_3$ | n = 0 | H | |
| 2.133 | $i-C_4H_9$ | $CH_3$ | 3-Cl | H | |
| 2.134 | $i-C_4H_9$ | $CH_3$ | 3-F | H | |
| 2.135 | $-CHFCH_3$ | Cl | n = 0 | H | |
| 2.136 | $-CHFCH_3$ | Cl | 3-Cl | H | |
| 2.137 | $-CHFCH_3$ | Cl | 3-F | H | |
| 2.138 | Prop-1-in-1-yl | Cl | n = 0 | H | |
| 2.139 | Prop-1-in-1-yl | Cl | 3-F | H | |
| 2.140 | Prop-1-in-1-yl | Cl | 3-Cl | H | |
| 2.141 | Prop-1-in-1-yl | $CH_3$ | n = 0 | H | |
| 2.142 | Prop-1-in-1-yl | $CH_3$ | 3-F | H | |
| 2.143 | But-1-in-1-yl | Cl | n = 0 | H | |
| 2.144 | But-1-in-1-yl | $CH_3$ | n = 0 | H | |
| 2.145 | But-1-in-1-yl | F | 3-Cl | H | |
| 2.146 | $OC_2H_5$ | Cl | n = 0 | H | |
| 2.147 | $OC_2H_5$ | Cl | 3-Cl | H | |
| 2.148 | $OC_2H_5$ | Cl | 3-F | H | |
| 2.149 | $OC_2H_5$ | $CH_3$ | 3-Cl | H | |
| 2.150 | cyclo-$C_3H_5$ | F | n = 0 | H | |
| 2.151 | cyclo-$C_3H_5$ | F | 3-Cl | H | |
| 2.152 | cyclo-$C_3H_5$ | F | 3-F | H | |
| 2.153 | cyclo-$C_3H_5$ | $CH_3$ | n = 0 | H | |
| 2.154 | cyclo-$C_3H_5$ | $CH_3$ | 3-Cl | H | |
| 2.155 | cyclo-$C_3H_5$ | $CH_3$ | 3-F | H | |
| 2.156 | cyclo-$C_3H_5$ | $CH_3$ | 3-F,5-F | H | |
| 2.157 | $OC_3H_7$ | Cl | n = 0 | H | |
| 2.158 | $OC_3H_7$ | Cl | 3-Cl | H | |
| 2.159 | $OC_3H_7$ | Cl | 3-F | H | |
| 2.160 | $OC_3H_7$-i | Cl | n = 0 | H | |

| Verb. Nr. | $R_1$ | $R_2$ | $(R_3)_n$ | $R_4$ | phys. Daten |
|---|---|---|---|---|---|
| 2.161 | $OC_3H_7$-i | Cl | 3-Cl | H | |
| 2.162 | $OC_3H_7$-i | Cl | 3-F | H | |
| 2.163 | cyclo-$C_4H_7$ | Cl | n = 0 | H | |
| 2.164 | cyclo-$C_4H_7$ | Cl | 3-Cl | H | |
| 2.165 | cyclo-$C_4H_7$ | F | n = 0 | H | |
| 2.166 | cyclo-$C_4H_7$ | F | 3-Cl | H | |
| 2.167 | cyclo-$C_5H_9$ | Cl | n = 0 | H | |
| 2.168 | cyclo-$C_5H_9$ | Cl | 3-Cl | H | |
| 2.169 | cyclo-$C_5H_9$ | Cl | 3-F | H | |
| 2.170 | cyclo-$C_5H_9$ | F | n = 0 | H | |
| 2.171 | cyclo-$C_5H_9$ | F | 3-Cl | H | |
| 2.172 | cyclo-$C_5H_9$ | F | 3-F | H | |
| 2.173 | cyclo-$C_6H_{11}$ | Cl | n = 0 | H | |
| 2.174 | cyclo-$C_6H_{11}$ | Cl | 3-Cl | H | |
| 2.175 | cyclo-$C_6H_{11}$ | Cl | 3-F | H | |
| 2.176 | $C_2H_5$ | Cl | n = 0 | $C_2H_5$ | |
| 2.177 | $C_2H_5$ | F | n = 0 | $C_2H_5$ | |
| 2.178 | $C_2H_5$ | Br | n = 0 | $C_2H_5$ | |
| 2.179 | $C_2H_5$ | Cl | 3-Cl | $C_2H_5$ | |
| 2.180 | $C_2H_5$ | Cl | 3-F | $C_2H_5$ | |
| 2.181 | $C_2H_5$ | Cl | n = 0 | $CH_3$ | |
| 2.182 | $C_3H_7$ | Br | 3-F,5-F | H | |
| 2.183 | $C_3H_7$ | Br | 3-Cl | H | |
| 2.184 | $R_1$+$R_4$: -$(CH_2)_3$- | Cl | n = 0 | | |
| 2.185 | $R_1$+$R_4$: -$(CH_2)_3$- | Cl | 3-Cl | | |
| 2.186 | $R_1$+$R_4$: -$(CH_2)_3$- | F | n = 0 | | |
| 2.187 | $R_1$+$R_4$: -$(CH_2)_3$- | F | 3-Cl | | |
| 2.188 | $R_1$+$R_4$: -$(CH_2)_3$- | F | 3-F | | |
| 2.189 | $R_1$+$R_4$: -$(CH_2)_3$- | $CH_3$ | n = 0 | | |
| 2.190 | $R_1$+$R_4$: -$(CH_2)_3$- | $CH_3$ | 3-Cl | | |
| 2.191 | $R_1$+$R_4$: -$(CH_2)_3$- | $CH_3$ | 3-F | | |
| 2.192 | $R_1$+$R_4$: -$(CH_2)_4$- | Cl | n = 0 | | |
| 2.193 | $R_1$+$R_4$: -$(CH_2)_4$- | F | n = 0 | | |
| 2.194 | $R_1$+$R_4$: -$(CH_2)_4$- | Br | n = 0 | | |

| Verb. Nr. | $R_1$ | $R_2$ | $(R_3)_n$ | $R_4$ | phys. Daten |
|---|---|---|---|---|---|
| 2.195 | $R_1$+$R_4$: -$(CH_2)_4$- | $CH_3$ | n = 0 | | |
| 2.196 | $R_1$+$R_4$: -$(CH_2)_4$- | $CH_3$ | 3-Cl | | |
| 2.197 | $C_3H_7$ | Br | n = 0 | H | |
| 2.198 | $R_1$+$R_4$: -$(CH_2)_5$- | Cl | n = 0 | | |
| 2.199 | $R_1$+$R_4$: -$(CH_2)_5$- | $CH_3$ | n = 0 | | |
| 2.200 | $R_1$+$R_4$: -$(CH_2)_5$- | F | n = 0 | | |
| 2.201 | $R_1$+$R_4$: -$(CH_2)_5$- | F | 3-Cl | | |
| 2.202 | $R_1$+$R_4$: -$CH_2CH_2CH$=CH- | Cl | n = 0 | | |
| 2.203 | $R_1$+$R_4$: -$CH_2CH_2CH$=CH- | Cl | 3-F | | |
| 2.204 | $R_1$+$R_4$: -$CH_2CH_2CH$=CH- | Cl | 3-Cl | | |
| 2.205 | $R_1$+$R_4$: -$CH_2OCH_2$- | Cl | n = 0 | | |
| 2.206 | $R_1$+$R_4$: -$CH_2OCH_2$- | Cl | 3-F | | |
| 2.207 | $R_1$+$R_4$: -$CH_2OCH_2$- | Cl | 3-Cl | | |
| 2.208 | $R_1$+$R_4$: -$CH_2OCH_2$- | F | n = 0 | | |
| 2.209 | $R_1$+$R_4$: -$CH_2OCH_2$- | F | 3-Cl | | |
| 2.210 | $R_1$+$R_4$: -$CH_2CH_2OCH_2CH_2$- | Cl | n = 0 | | |
| 2.211 | $R_1$+$R_4$: -$CH_2CH_2OCH_2CH_2$- | Cl | 3-Cl | | |
| 2.212 | $R_1$+$R_4$: -$CH_2CH_2OCH_2CH_2$- | Cl | 3-F | | |
| 2.213 | $R_1$+$R_4$: -$CH_2CH(CH_3)OCH_2CH_2$- | Cl | n = 0 | | |
| 2.214 | $R_1$+$R_4$: -$CH_2CH(CH_3)OCH_2CH_2$- | Cl | 3-Cl | | |
| 2.215 | $C_2H_5$ | $C_2H_5$ | n = 0 | H | |

| Verb. Nr. | $R_1$ | $R_2$ | $(R_3)_n$ | $R_4$ | phys. Daten |
|---|---|---|---|---|---|
| 2.216 | $C_2H_5$ | $C_2H_5$ | 3-Cl | H | |
| 2.217 | $C_2H_5$ | $C_2H_5$ | 3-F | H | |
| 2.218 | $C_2H_5$ | $C_2H_5$ | 3-F,5-F | H | |
| 2.219 | $C_2H_5$ | $C_3H_7$ | n = 0 | H | |
| 2.220 | $C_2H_5$ | $C_3H_7$ | 3-Cl | H | |
| 2.221 | $C_2H_5$ | $C_3H_7$ | 3-F | H | |
| 2.222 | $C_2H_5$ | i-$C_3H_7$ | n = 0 | H | |
| 2.223 | $C_2H_5$ | i-$C_3H_7$ | 3-Cl | H | |
| 2.224 | $C_2H_5$ | i-$C_3H_7$ | 3-F | H | |
| 2.225 | $C_3H_7$ | i-$C_3H_7$ | n = 0 | H | |
| 2.226 | $C_3H_7$ | i-$C_3H_7$ | 3-Cl | H | |
| 2.227 | $C_2H_5$ | $CF_3$ | n = 0 | H | |
| 2.228 | $C_2H_5$ | $CF_3$ | 3-Cl | H | |
| 2.229 | $C_2H_5$ | $CF_3$ | 3-F | H | |
| 2.230 | $C_3H_7$ | $CF_3$ | n = 0 | H | |
| 2.231 | $C_3H_7$ | $CF_3$ | 3-Cl | H | |
| 2.232 | $C_3H_7$ | $CF_3$ | 3-F | H | |
| 2.233 | $CH_3$ | $OCH_3$ | n = 0 | H | |
| 2.234 | $CH_3$ | $OCH_3$ | 3-Cl | H | |
| 2.235 | $C_2H_5$ | $OCH_3$ | n = 0 | H | |
| 2.236 | $C_2H_5$ | $OCH_3$ | 3-Cl | H | |
| 2.237 | $C_2H_5$ | $OCH_3$ | 3-F | H | |
| 2.238 | $C_3H_7$ | $OCH_3$ | n = 0 | H | |
| 2.239 | $C_3H_7$ | $OCH_3$ | 3-Cl | H | |
| 2.240 | $C_3H_7$ | $OCH_3$ | 3-F,5-F | H | |
| 2.241 | Ethinyl | Cl | n = 0 | H | |
| 2.242 | Ethinyl | Cl | 3-F | H | |
| 2.243 | Ethinyl | Cl | 3-F,5-F | H | |
| 2.244 | $C_2H_5$ | Br | 3-F,5-F | H | |
| 2.245 | $CH_3$ | $OCF_3$ | n = 0 | $CH_3$ | |
| 2.246 | $CH_3$ | $OCF_3$ | n = 0 | H | |
| 2.247 | $CH_3$ | $OCF_3$ | 3-Cl | H | |
| 2.248 | $C_2H_5$ | $OCF_3$ | n = 0 | H | |
| 2.249 | $C_2H_5$ | $OCF_3$ | 3-Cl | H | |

| Verb. Nr. | $R_1$ | $R_2$ | $(R_3)_n$ | $R_4$ | phys. Daten |
|---|---|---|---|---|---|
| 2.250 | $C_2H_5$ | $OCF_3$ | 3-F | H | |
| 2.251 | $C_2H_5$ | $OCF_3$ | 3-F,5-F | H | |
| 2.252 | $C_3H_7$ | $OCF_3$ | n = 0 | H | |
| 2.253 | $C_3H_7$ | $OCF_3$ | 3-Cl | H | |
| 2.254 | $C_3H_7$ | $OCF_3$ | 3-F | H | |
| 2.255 | $CH_3$ | F | n = 0 | $CH_3$ | |
| 2.256 | $CH_3$ | F | n = 0 | H | |
| 2.257 | $CH_3$ | F | 3-F | H | |
| 2.258 | $CH_3$ | F | 3-Cl | H | |
| 2.259 | $C_2H_5$ | F | 3-F,5-F | H | |
| 2.260 | $C_2H_5$ | F | 3-$CH_3$ | H | |
| 2.261 | $C_2H_5$ | F | 4-$CH_3$ | H | |
| 2.262 | $C_2H_5$ | F | n = 0 | H | |
| 2.263 | $C_3H_7$ | F | 3-F,5-F | H | |
| 2.264 | $C_3H_7$ | F | 3-$CH_3$ | H | |
| 2.265 | $C_3H_7$ | F | 4-$CH_3$ | H | |
| 2.266 | $C_2H_5$ | Br | 3-Cl | H | |
| 2.267 | $CH_3$ | Br | n = 0 | $CH_3$ | |
| 2.268 | $CH_3$ | Br | n = 0 | H | |
| 2.269 | $CH_3$ | Br | 3-Cl | H | |
| 2.270 | $CH_3$ | Br | 3-F | H | |
| 2.271 | -CH=CH$CH_3$ (E/Z) | F | n = 0 | H | |

Tabelle 3

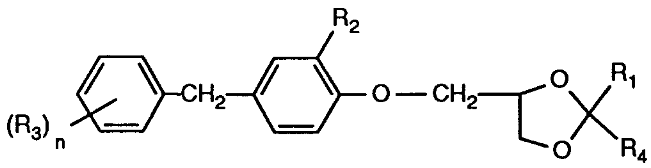

| Verb. Nr. | $R_1$ | $R_2$ | $(R_3)_n$ | $R_4$ | phys. Daten |
|---|---|---|---|---|---|
| 3.1 | $CH_3$ | Cl | n = 0 | $CH_3$ | $n_D^{20} = 1.5554$ |
| 3.2 | $CH_3$ | Cl | n = 0 | H | |
| 3.3 | $C_2H_5$ | Cl | n = 0 | H | |
| 3.3.1 | $C_2H_5$ | Cl | n = 0 | H | A; $n_D^{20} = 1.5573$ |
| 3.3.2 | $C_2H_5$ | Cl | n = 0 | H | B; $n_D^{20} = 1.5587$ |
| 3.4 | $C_3H_7$ | Cl | n = 0 | H | |
| 3.4.1 | $C_3H_7$ | Cl | n = 0 | H | A; $n_D^{20} = 1.5524$ |
| 3.4.2 | $C_3H_7$ | Cl | n = 0 | H | B; $n_D^{20} = 1.5533$ |
| 3.5 | i-$C_3H_7$ | Cl | n = 0 | H | |
| 3.5.1 | i-$C_3H_7$ | Cl | n = 0 | H | A; $n_D^{20} = 1.5512$ |
| 3.5.2 | i-$C_3H_7$ | Cl | n = 0 | H | B; $n_D^{20} = 1.5522$ |
| 3.6 | cyclo-$C_3H_5$ | Cl | n = 0 | H | |
| 3.6.1 | cyclo-$C_3H_5$ | Cl | n = 0 | H | A; $n_D^{20} = 1.5670$ |
| 3.6.2 | cyclo-$C_3H_5$ | Cl | n = 0 | H | B; $n_D^{20} = 1.5682$ |
| 3.7 | i-$C_4H_9$ | Cl | n = 0 | H | |
| 3.8 | $C_2H_5$ | $CH_3$ | n = 0 | H | |
| 3.9 | i-$C_3H_7$ | $CH_3$ | n = 0 | H | |
| 3.10 | cyclo-$C_3H_5$ | $CH_3$ | n = 0 | H | |
| 3.11 | $CH_3$ | Cl | 3-F | $CH_3$ | |
| 3.12 | $CH_3$ | Cl | 3-F | H | |
| 3.12.1 | $CH_3$ | Cl | 3-F | H | A; $n_D^{20} = 1.5530$ |
| 3.12.2 | $CH_3$ | Cl | 3-F | H | B; $n_D^{20} = 1.5549$ |
| 3.13 | $C_2H_5$ | Cl | 3-F | H | |
| 3.13.1 | $C_2H_5$ | Cl | 3-F | H | A; $n_D^{20} = 1.5481$ |
| 3.13.2 | $C_2H_5$ | Cl | 3-F | H | B; $n_D^{20} = 1.5490$ |
| 3.14 | $C_3H_7$ | Cl | 3-F | H | |
| 3.14.1 | $C_3H_7$ | Cl | 3-F | H | A; $n_D^{20} = 1.5439$ |
| 3.14.2 | $C_3H_7$ | Cl | 3-F | H | B; $n_D^{20} = 1.5452$ |
| 3.15 | i-$C_3H_7$ | Cl | 3-F | H | |

| Verb. Nr. | $R_1$ | $R_2$ | $(R_3)_n$ | $R_4$ | phys. Daten |
|---|---|---|---|---|---|
| 3.16 | cyclo-$C_3H_5$ | Cl | 3-F | H | |
| 3.17 | i-$C_4H_9$ | Cl | 3-F | H | |
| 3.18 | $C_4H_9$ | $CH_3$ | 3-F | H | |
| 3.19 | $CH_3$ | Cl | 3-F,5-F | $CH_3$ | |
| 3.20 | $CH_3$ | Cl | 3-F,5-F | H | |
| 3.21 | $C_2H_5$ | Cl | 3-F,5-F | H | |
| 3.21.1 | $C_2H_5$ | Cl | 3-F,5-F | H | A; $n_D^{20}$ = 1.5401 |
| 3.21.2 | $C_2H_5$ | Cl | 3-F,5-F | H | B; $n_D^{20}$ = 1.5409 |
| 3.22 | $C_3H_7$ | Cl | 3-F,5-F | H | |
| 3.23 | i-$C_3H_7$ | Cl | 3-F,5-F | H | |
| 3.24 | cyclo-$C_3H_5$ | Cl | 3-F,5-F | H | |
| 3.25 | i-$C_4H_9$ | Cl | 3-F,5-F | H | |
| 3.26 | $C_2H_5$ | Cl | 3-Cl,4-Cl | H | |
| 3.26.1 | $C_2H_5$ | Cl | 3-Cl,4-Cl | H | A; Smp. = 86-87° |
| 3.26.2 | $C_2H_5$ | Cl | 3-Cl,4-Cl | H | B; Smp. = 66-67° |
| 3.27 | $C_3H_7$ | Cl | 3-Cl,4-Cl | H | |
| 3.27.1 | $C_3H_7$ | Cl | 3-Cl,4-Cl | H | A; Smp. = 52-53° |
| 3.27.2 | $C_3H_7$ | Cl | 3-Cl,4-Cl | H | B; Smp. = 58-59° |
| 3.28 | cyclo-$C_3H_5$ | Cl | 3-Cl,4-Cl | H | |
| 3.28.1 | cyclo-$C_3H_5$ | Cl | 3-Cl,4-Cl | H | A; $n_D^{20}$ = 1.5808 |
| 3.28.2 | cyclo-$C_3H_5$ | Cl | 3-Cl,4-Cl | H | B; $n_D^{20}$ = 1.5820 |
| 3.29 | $C_2H_5$ | Br | 3-Cl,4-Cl | H | Smp. = 70-72° |
| 3.30 | $C_2H_5$ | Br | n = 0 | H | |
| 3.30.1 | $C_2H_5$ | Br | n = 0 | H | A; $n_D^{20}$ = 1.5701 |
| 3.30.2 | $C_2H_5$ | Br | n = 0 | H | B; $n_D^{20}$ = 1.5718 |
| 3.31 | i-$C_3H_7$ | Br | n = 0 | H | |
| 3.31.1 | i-$C_3H_7$ | Br | n = 0 | H | A; $n_D^{20}$ = 1.5630 |
| 3.31.2 | i-$C_3H_7$ | Br | n = 0 | H | B; $n_D^{20}$ = 1.5641 |
| 3.32 | $C_2H_5$ | F | 3-F | H | |
| 3.32.1 | $C_2H_5$ | F | 3-F | H | A; $n_D^{20}$ = 1.5304 |
| 3.32.2 | $C_2H_5$ | F | 3-F | H | B; $n_D^{20}$ = 1.5311 |
| 3.33 | $C_3H_7$ | F | 3-F | H | |
| 3.33.1 | $C_3H_7$ | F | 3-F | H | A; Smp. = 47-48° |
| 3.33.2 | $C_3H_7$ | F | 3-F | H | B; $n_D^{20}$ = 1.5269 |

| Verb. Nr. | $R_1$ | $R_2$ | $(R_3)_n$ | $R_4$ | phys. Daten |
|---|---|---|---|---|---|
| 3.34 | Ethinyl | Cl | n = 0 | H | $n_D^{20} = 1.5695$ |
| 3.35 | $CH_3$ | Cl | 3-$CF_3$ | $CH_3$ | |
| 3.36 | $CH_3$ | Cl | 3-Cl | H | |
| 3.37 | $CH_3$ | Cl | 3-$CH_3$ | H | |
| 3.38 | $C_2H_5$ | Cl | 3-F,4-Cl | H | |
| 3.39 | $C_2H_5$ | Cl | 3-Cl | H | |
| 3.40 | $C_2H_5$ | Cl | 3-$CH_3$ | H | |
| 3.41 | $C_2H_5$ | Cl | 3-$OCH_3$ | H | |
| 3.42 | $C_2H_5$ | Cl | 3-$CF_3$ | H | |
| 3.43 | $C_2H_5$ | Cl | 3-$OCF_3$ | H | |
| 3.44 | $C_2H_5$ | Cl | 4-F | H | |
| 3.45 | $C_2H_5$ | Cl | 3-Cl,5-Cl | H | |
| 3.46 | $C_3H_7$ | Cl | 3-Cl | H | |
| 3.47 | $C_3H_7$ | Cl | 3-$CH_3$ | H | |
| 3.48 | $C_3H_7$ | Cl | 3-$C_2H_5$ | H | |
| 3.49 | $C_3H_7$ | Cl | 3-$OCH_3$ | H | |
| 3.50 | $C_3H_7$ | Cl | 3-$CF_3$ | H | |
| 3.51 | $C_3H_7$ | Cl | 3-$OCF_3$ | H | |
| 3.52 | $C_3H_7$ | Cl | 4-F | H | |
| 3.53 | i-$C_3H_7$ | Cl | 3-Cl | H | |
| 3.54 | i-$C_3H_7$ | Cl | 3-$CH_3$ | H | |
| 3.55 | i-$C_3H_7$ | Cl | 3-$C_2H_5$ | H | |
| 3.56 | $C_2H_5$ | F | n = 0 | H | |
| 3.57 | $C_2H_5$ | F | 3-Cl | H | |
| 3.58 | $C_2H_5$ | F | 3-F,5-F | H | |
| 3.59 | $C_3H_7$ | F | n = 0 | H | |
| 3.60 | $C_3H_7$ | F | 3-Cl | H | |
| 3.61 | cyclo-$C_3H_5$ | Cl | 3-Cl | H | |
| 3.62 | cyclo-$C_3H_5$ | Cl | 3-$CH_3$ | H | |
| 3.63 | i-$C_4H_9$ | Cl | 3-Cl | H | |
| 3.64 | i-$C_4H_9$ | Cl | 3-$CH_3$ | H | |
| 3.65 | $C_6H_{13}$ | Cl | n = 0 | H | |
| 3.66 | $C_6H_{13}$ | Cl | 3-Cl | H | |
| 3.67 | $-CH=CH_2$ | Cl | n = 0 | H | |

| Verb. Nr. | $R_1$ | $R_2$ | $(R_3)_n$ | $R_4$ | phys. Daten |
|---|---|---|---|---|---|
| 3.68 | $-CH=CH_2$ | Cl | 3-Cl | H | |
| 3.69 | $-CH=CH_2$ | Cl | 3-F | H | |
| 3.70 | $-CH=CH_2$ | Cl | 3-F,5-F | H | |
| 3.71 | Ethinyl | Cl | 3-Cl | H | |
| 3.72 | Ethinyl | Cl | 3-F | H | |
| 3.73 | Ethinyl | Cl | 3-F,5-F | H | |
| 3.74 | Prop-1-in-1-yl | Cl | n = 0 | H | |
| 3.75 | Prop-1-in-1-yl | Cl | 3-Cl | H | |
| 3.76 | Prop-1-in-1-yl | Cl | 3-F | H | |
| 3.77 | Prop-1-en-1-yl | Cl | n = 0 | H | |
| 3.78 | Prop-1-en-1-yl | Cl | 3-Cl | H | |
| 3.79 | Prop-1-en-1-yl | Cl | 3-F | H | |
| 3.80 | $-CH=C(CH_3)_2$ | Cl | n = 0 | H | |
| 3.81 | $-CH=C(CH_3)_2$ | Cl | 3-Cl | H | |
| 3.82 | $-CH=C(CH_3)_2$ | Cl | 3-F | H | |
| 3.83 | $CH_3$ | $CH_3$ | n = 0 | $CH_3$ | |
| 3.84 | $CH_3$ | $CH_3$ | 3-Cl | $CH_3$ | |
| 3.85 | $CH_3$ | $CH_3$ | n = 0 | H | |
| 3.86 | $CH_3$ | $CH_3$ | 3-Cl | H | |
| 3.87 | $CH_3$ | $CH_3$ | 3-F | H | |
| 3.88 | $C_2H_5$ | $CH_3$ | 3-Cl | H | |
| 3.89 | $C_2H_5$ | $CH_3$ | 3-F | H | |
| 3.90 | $C_2H_5$ | $CH_3$ | 3-F,5-F | H | |
| 3.91 | $C_2H_5$ | $CH_3$ | $3-C_2H_5$ | H | |
| 3.92 | $C_3H_7$ | $CH_3$ | n = 0 | H | |
| 3.93 | $C_3H_7$ | $CH_3$ | 3-Cl | H | |
| 3.94 | $C_3H_7$ | $CH_3$ | 3-F | H | |
| 3.95 | $C_3H_7$ | $CH_3$ | 3-F,5-F | H | |
| 3.96 | $C_3H_7$ | $CH_3$ | $3-CH_3$ | H | |
| 3.97 | $i-C_3H_7$ | $CH_3$ | 3-Cl | H | |
| 3.98 | $i-C_3H_7$ | $CH_3$ | 3-F | H | |
| 3.99 | $cyclo-C_3H_5$ | $CH_3$ | 3-Cl | H | |
| 3.100 | $cyclo-C_3H_5$ | $CH_3$ | 3-F | H | |
| 3.101 | $-CH=CH_2$ | $CH_3$ | n = 0 | H | |

| Verb. Nr. | $R_1$ | $R_2$ | $(R_3)_n$ | $R_4$ | phys. Daten |
|---|---|---|---|---|---|
| 3.102 | $-CH=CH_2$ | $CH_3$ | 3-Cl | H | |
| 3.103 | $-CH=CH_2$ | $CH_3$ | 3-F | H | |
| 3.104 | Ethinyl | $CH_3$ | n = 0 | H | |
| 3.105 | Ethinyl | $CH_3$ | 3-Cl | H | |
| 3.106 | Ethinyl | $CH_3$ | 3-F | H | |
| 3.107 | $R_1$+$R_4$: $-(CH_2)_3-$ | Cl | n = 0 | | |
| 3.108 | $R_1$+$R_4$: $-(CH_2)_3-$ | Cl | 3-F | | |
| 3.109 | $R_1$+$R_4$: $-(CH_2)_3-$ | Cl | 3-Cl | | |
| 3.110 | $R_1$+$R_4$: $-(CH_2)_4-$ | Cl | n = 0 | | |
| 3.111 | $R_1$+$R_4$: $-(CH_2)_4-$ | Cl | 3-F | | |
| 3.112 | $R_1$+$R_4$: $-(CH_2)_4-$ | Cl | 3-Cl | | |
| 3.113 | $R_1$+$R_4$: $-(CH_2)_5-$ | Cl | n = 0 | | |
| 3.114 | $R_1$+$R_4$: $-(CH_2)_5-$ | Cl | 3-F | | |
| 3.115 | $R_1$+$R_4$: $-(CH_2)_5-$ | Cl | $3-CH_3$ | | |
| 3.116 | $R_1$+$R_4$: $-CH_2OCH_2CH_2CH_2-$ | Cl | n = 0 | | |
| 3.117 | $R_1$+$R_4$: $-CH_2OCH_2CH_2CH_2-$ | Cl | 3-F | | |
| 3.118 | $R_1$+$R_4$: $-CH_2OCH_2CH_2CH_2-$ | Cl | 3-Cl | | |
| 3.119 | $C_2H_5$ | $CF_3$ | n = 0 | H | |
| 3.120 | $C_2H_5$ | $CF_3$ | 3-Cl | H | |
| 3.121 | $C_2H_5$ | $CF_3$ | 3-F | H | |
| 3.122 | $C_3H_7$ | $CF_3$ | n = 0 | H | |
| 3.123 | $C_3H_7$ | $CF_3$ | 3-Cl | H | |
| 3.124 | $C_3H_7$ | $CF_3$ | 3-F | H | |
| 3.125 | $C_2H_5$ | $OCH_3$ | n = 0 | H | |
| 3.126 | $C_2H_5$ | $OCH_3$ | 3-Cl | H | |
| 3.127 | $C_2H_5$ | $OCH_3$ | 3-F | H | |
| 3.128 | $OCH_3$ | Cl | n = 0 | $CH_3$ | |
| 3.129 | $OCH_3$ | Cl | 3-Cl | $CH_3$ | |
| 3.130 | $OCH_3$ | Cl | 3-F | $CH_3$ | |
| 3.131 | $OCH_3$ | Cl | 3-F,5-F | H | |
| 3.132 | $OC_2H_5$ | Cl | n = 0 | H | |

| Verb. Nr. | $R_1$ | $R_2$ | $(R_3)_n$ | $R_4$ | phys. Daten |
|---|---|---|---|---|---|
| 3.133 | $OC_2H_5$ | Cl | 3-Cl | H | |
| 3.134 | $OC_2H_5$ | Cl | 3-F | H | |
| 3.135 | $OC_2H_5$ | Cl | 3-F,5-F | H | |
| 3.136 | $OC_2H_5$ | Cl | 3-$CH_3$ | H | |
| 3.137 | $OCH(CH_3)_2$ | Cl | n = 0 | H | |
| 3.138 | $OCH(CH_3)_2$ | Cl | 3-Cl | H | |
| 3.139 | $OCH(CH_3)_2$ | Cl | 3-F | H | |
| 3.140 | $OCH_2CH_2CH_3$ | Cl | n = 0 | H | |
| 3.141 | $OCH_2CH_2CH_3$ | Cl | 3-Cl | H | |
| 3.142 | $OCH_2CH_2CH_3$ | Cl | 3-F | H | |
| 3.143 | $OCH_3$ | Br | n = 0 | H | |
| 3.144 | $OCH_3$ | Br | 3-Cl | H | |
| 3.145 | $OCH_3$ | Br | 3-F | H | |
| 3.146 | $OC_2H_5$ | Br | n = 0 | H | |
| 3.147 | $OC_2H_5$ | Br | 3-Cl | H | |
| 3.148 | $OC_2H_5$ | Br | 3-Br | H | |
| 3.149 | $OCH_3$ | F | n = 0 | H | |
| 3.150 | $OCH_3$ | F | 3-Cl | H | |
| 3.151 | $OCH_3$ | F | 3-F | H | |
| 3.152 | $OC_2H_5$ | F | n = 0 | H | |
| 3.153 | $OC_2H_5$ | F | 3-Cl | H | |
| 3.154 | $OC_2H_5$ | F | 3-Br | H | |
| 3.155 | $OCH_3$ | $CH_3$ | n = 0 | H | |
| 3.156 | $OCH_3$ | $CH_3$ | 3-Cl | H | |
| 3.157 | $OCH_3$ | $CH_3$ | 3-Br | H | |
| 3.158 | $OCH_3$ | $CH_3$ | 3-F | H | |
| 3.159 | $OC_2H_5$ | $CH_3$ | n = 0 | H | |
| 3.160 | $OC_2H_5$ | $CH_3$ | 3-Cl | H | |
| 3.161 | $OC_2H_5$ | $CH_3$ | 3-F | H | |

Tabelle 4

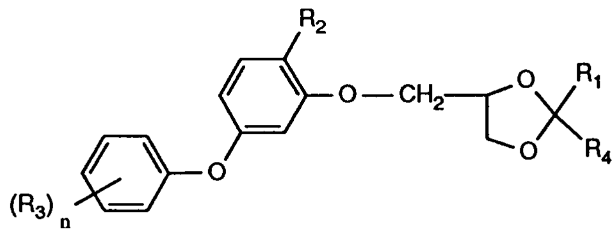

| Verb. Nr. | $R_1$ | $R_2$ | $(R_3)_n$ | $R_4$ | phys. Daten |
|---|---|---|---|---|---|
| 4.1 | $C_2H_5$ | Cl | 3-Cl | H | $n_D^{20} = 1.5662$ |
| 4.2 | $C_3H_7$ | Cl | 3-Cl | H | $n_D^{20} = 1.5609$ |
| 4.3 | $CH_3$ | Cl | n = 0 | $CH_3$ | |
| 4.4 | $CH_3$ | Cl | n = 0 | H | |
| 4.5 | $CH_3$ | Cl | 3-Cl | H | |
| 4.6 | $CH_3$ | Cl | 3-F | H | |
| 4.7 | $C_2H_5$ | Cl | n = 0 | H | |
| 4.8 | $C_2H_5$ | Cl | 3-F | H | |
| 4.9 | $C_2H_5$ | Cl | 3-F,5-F | H | |
| 4.10 | $C_2H_5$ | Cl | 3-$CH_3$ | H | |
| 4.11 | $C_3H_7$ | Cl | n = 0 | H | |
| 4.12 | $C_3H_7$ | Cl | 3-F | H | |
| 4.13 | $C_3H_7$ | Cl | 3-F,5-F | H | |
| 4.14 | i-$C_3H_7$ | Cl | n = 0 | H | |
| 4.15 | i-$C_3H_7$ | Cl | 3-Cl | H | |
| 4.16 | i-$C_3H_7$ | Cl | 3-F | H | |
| 4.17 | cyclo-$C_3H_5$ | Cl | n = 0 | H | |
| 4.18 | cyclo-$C_3H_5$ | Cl | 3-F | H | |
| 4.19 | cyclo-$C_3H_5$ | Cl | 3-Cl | H | |
| 4.20 | cyclo-$C_3H_5$ | Cl | 3-F,5-F | H | |
| 4.21 | $C_2H_5$ | Br | n = 0 | H | |
| 4.22 | $C_2H_5$ | Br | 3-Cl | H | |
| 4.23 | $C_2H_5$ | Br | 3-F | H | |
| 4.24 | $C_3H_7$ | Br | n = 0 | H | |
| 4.25 | $C_3H_7$ | Br | 3-Cl | H | |
| 4.26 | $C_3H_7$ | Br | 3-F | H | |
| 4.27 | $C_2H_5$ | $CH_3$ | n = 0 | H | |

| Verb. Nr. | $R_1$ | $R_2$ | $(R_3)_n$ | $R_4$ | phys. Daten |
|---|---|---|---|---|---|
| 4.28 | $C_2H_5$ | $CH_3$ | 3-Cl | H | |
| 4.29 | $C_2H_5$ | $CH_3$ | 3-F | H | |
| 4.30 | $C_2H_5$ | $CH_3$ | 3-F,5-F | H | |
| 4.31 | $C_3H_7$ | $CH_3$ | n = 0 | H | |
| 4.32 | $C_3H_7$ | $CH_3$ | 3-Cl | H | |
| 4.33 | $C_3H_7$ | $CH_3$ | 3-F | H | |

Tabelle 5

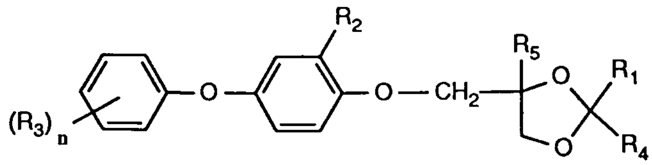

| Verb. Nr. | $R_1$ | $R_2$ | $(R_3)_n$ | $R_4$ | $R_5$ | phys. Daten |
|---|---|---|---|---|---|---|
| 5.1 | $C_2H_5$ | Cl | 3-F | H | $CH_3$ | |
| 5.2 | $CH_3$ | Cl | n = 0 | H | $CH_3$ | |
| 5.3 | $CH_3$ | Cl | n = 0 | $CH_3$ | $CH_3$ | |
| 5.4 | $CH_3$ | Cl | 3-Cl | H | $CH_3$ | |
| 5.5 | $CH_3$ | Cl | 3-F | H | $CH_3$ | |
| 5.6 | $C_2H_5$ | Cl | n = 0 | H | $CH_3$ | |
| 5.7 | $C_2H_5$ | Cl | 3-Cl | H | $CH_3$ | |
| 5.8 | $C_2H_5$ | Cl | 3-F,5-F | H | $CH_3$ | |
| 5.9 | $C_3H_7$ | Cl | n = 0 | H | $CH_3$ | |
| 5.10 | $C_3H_7$ | Cl | 3-Cl | H | $CH_3$ | |
| 5.11 | $C_3H_7$ | Cl | 3-F | H | $CH_3$ | |
| 5.12 | $C_3H_7$ | Cl | 3-F,5-F | H | $CH_3$ | |
| 5.13 | cyclo-$C_3H_5$ | Cl | n = 0 | H | $CH_3$ | |
| 5.14 | cyclo-$C_3H_5$ | Cl | 3-Cl | H | $CH_3$ | |
| 5.15 | cyclo-$C_3H_5$ | Cl | 3-F | H | $CH_3$ | |
| 5.16 | i-$C_3H_7$ | Cl | n = 0 | H | $CH_3$ | |
| 5.17 | i-$C_3H_7$ | Cl | 3-Cl | H | $CH_3$ | |
| 5.18 | i-$C_3H_7$ | Cl | 3-F | H | $CH_3$ | |
| 5.19 | $C_2H_5$ | F | n = 0 | H | $CH_3$ | |
| 5.20 | $C_2H_5$ | F | 3-Cl | H | $CH_3$ | |
| 5.21 | $C_2H_5$ | F | 3-F | H | $CH_3$ | |
| 5.22 | $C_2H_5$ | F | 3-F,5-F | H | $CH_3$ | |
| 5.23 | $C_3H_7$ | F | n = 0 | H | $CH_3$ | |
| 5.24 | $C_3H_7$ | F | 3-Cl | H | $CH_3$ | |
| 5.25 | $C_3H_7$ | F | 3-F | H | $CH_3$ | |
| 5.26 | i-$C_4H_9$ | F | n = 0 | H | $CH_3$ | |
| 5.27 | i-$C_4H_9$ | F | 3-Cl | H | $CH_3$ | |
| 5.28 | $C_2H_5$ | $CH_3$ | n = 0 | H | $CH_3$ | |
| 5.29 | $C_2H_5$ | $CH_3$ | 3-Cl | H | $CH_3$ | |

| Verb. Nr. | $R_1$ | $R_2$ | $(R_3)_n$ | $R_4$ | $R_5$ | phys. Daten |
|---|---|---|---|---|---|---|
| 5.30 | $C_2H_5$ | $CH_3$ | 3-F | H | $CH_3$ | |
| 5.31 | $C_2H_5$ | $CH_3$ | 3-F,5-F | H | $CH_3$ | |
| 5.32 | $C_3H_7$ | $CH_3$ | n = 0 | H | $CH_3$ | |
| 5.33 | $C_3H_7$ | $CH_3$ | 3-Cl | H | $CH_3$ | |
| 5.34 | $C_3H_7$ | $CH_3$ | 3-F | H | $CH_3$ | |
| 5.35 | $C_3H_7$ | $CH_3$ | 3-F,5-F | H | $CH_3$ | |
| 5.36 | $C_2H_5$ | Cl | n = 0 | H | $C_2H_5$ | |
| 5.37 | $C_2H_5$ | Cl | 3-Cl | H | $C_2H_5$ | |
| 5.38 | $C_2H_5$ | Cl | 3-F | H | $C_2H_5$ | |
| 5.39 | $C_2H_5$ | Cl | n = 0 | H | $C_3H_7$ | |
| 5.40 | $C_2H_5$ | Cl | 3-Cl | H | $C_3H_7$ | |
| 5.41 | $C_2H_5$ | Cl | 3-F | H | $C_3H_7$ | |

Tabelle 6

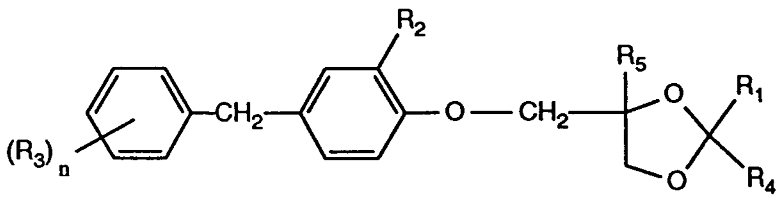

| Verb. Nr. | $R_1$ | $R_2$ | $(R_3)_n$ | $R_4$ | $R_5$ | phys. Daten |
|---|---|---|---|---|---|---|
| 6.1 | $C_3H_7$ | $CH_3$ | 3-F | H | $CH_3$ | |
| 6.2 | $C_3H_7$ | $CH_3$ | 3-Cl | H | $CH_3$ | |
| 6.3 | $CH_3$ | Cl | n = 0 | $CH_3$ | $CH_3$ | |
| 6.4 | $CH_3$ | Cl | n = 0 | H | $CH_3$ | |
| 6.5 | $C_2H_5$ | Cl | n = 0 | H | $CH_3$ | |
| 6.6 | $C_2H_5$ | Cl | 3-Cl | H | $CH_3$ | |
| 6.7 | $C_2H_5$ | Cl | 3-F | H | $CH_3$ | |
| 6.8 | $C_2H_5$ | Cl | 3-F,5-F | H | $CH_3$ | |
| 6.9 | $C_2H_5$ | $CH_3$ | n = 0 | H | $CH_3$ | |
| 6.10 | $C_2H_5$ | $CH_3$ | 3-Cl | H | $CH_3$ | |
| 6.11 | $C_2H_5$ | $CH_3$ | 3-F | H | $CH_3$ | |
| 6.12 | $C_2H_5$ | Br | n = 0 | H | $CH_3$ | |
| 6.13 | $C_2H_5$ | Br | 3-Cl | H | $CH_3$ | |
| 6.14 | $C_2H_5$ | Br | 3-F | H | $CH_3$ | |
| 6.15 | $C_2H_5$ | F | n = 0 | H | $CH_3$ | |
| 6.16 | $C_2H_5$ | F | 3-Cl | H | $CH_3$ | |
| 6.17 | $C_2H_5$ | F | 3-F | H | $CH_3$ | |
| 6.18 | $C_2H_5$ | F | 4-F | H | $CH_3$ | |
| 6.19 | $C_2H_5$ | F | 3-F,5-F | H | $CH_3$ | |
| 6.20 | $C_3H_7$ | Cl | n = 0 | H | $CH_3$ | |
| 6.21 | $C_3H_7$ | Cl | 3-Cl | H | $CH_3$ | |
| 6.22 | $C_3H_7$ | Cl | 3-F | H | $CH_3$ | |
| 6.23 | $C_3H_7$ | Cl | 3-F,5-F | H | $CH_3$ | |
| 6.24 | $C_3H_7$ | $CH_3$ | n = 0 | H | $CH_3$ | |

Tabelle 7

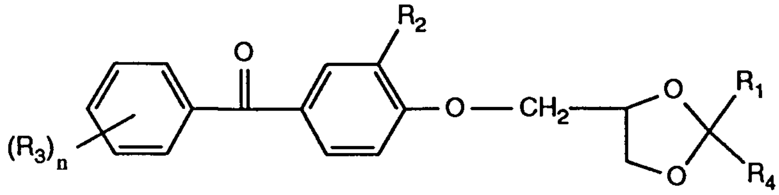

| Verb. Nr. | $R_1$ | $R_2$ | $(R_3)_n$ | $R_4$ | phys. Daten |
|---|---|---|---|---|---|
| 7.1 | cyclo-$C_3H_5$ | Cl | n = 0 | H | |
| 7.1.1 | cyclo-$C_3H_5$ | Cl | n = 0 | H | A; $n_D^{20}$ = 1.5825 |
| 7.1.2 | cyclo-$C_3H_5$ | Cl | n = 0 | H | B; $n_D^{20}$ = 1.5859 |
| 7.2 | $C_2H_5$ | Cl | n = 0 | H | $n_D^{20}$ = 1.5811 |
| 7.3 | $C_3H_7$ | Cl | 4-Cl | H | |
| 7.3.1 | $C_3H_7$ | Cl | 4-Cl | H | A; Smp. = 74-75° |
| 7.3.2 | $C_3H_7$ | Cl | 4-Cl | H | B; Smp. = 83-84° |
| 7.4 | $C_2H_5$ | Cl | 4-Cl | H | |
| 7.4.1 | $C_2H_5$ | Cl | 4-Cl | H | A; Smp. = 100-101° |
| 7.4.2 | $C_2H_5$ | Cl | 4-Cl | H | B; Smp. = 101-102° |
| 7.5 | $CH_3$ | Cl | n = 0 | $CH_3$ | |
| 7.6 | $CH_3$ | Cl | n = 0 | H | |
| 7.7 | $CH_3$ | Cl | 3-Cl | H | |
| 7.8 | $CH_3$ | Cl | 3-F | H | |
| 7.9 | $C_2H_5$ | Cl | 3-Cl | H | |
| 7.10 | $C_2H_5$ | Cl | 3-F | H | |
| 7.11 | $C_3H_7$ | Cl | 3-Cl | H | |
| 7.12 | $C_3H_7$ | Cl | 3-F | H | |
| 7.13 | $C_2H_5$ | Br | n = 0 | H | |
| 7.14 | $C_2H_5$ | Br | 3-Cl | H | |
| 7.15 | $C_2H_5$ | Br | 3-F | H | |

Tabelle 8

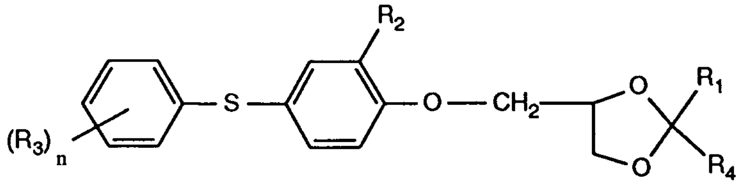

| Verb. Nr. | $R_1$ | $R_2$ | $(R_3)_n$ | $R_4$ | phys. Daten |
|---|---|---|---|---|---|
| 8.1 | $CH_3$ | Cl | n = 0 | $CH_3$ | |
| 8.2 | $CH_3$ | Cl | 3-Cl | $CH_3$ | |
| 8.3 | $CH_3$ | Cl | 3-F | $CH_3$ | |
| 8.4 | $CH_3$ | Cl | n = 0 | H | |
| 8.5 | $CH_3$ | Cl | 3-Cl | H | |
| 8.6 | $CH_3$ | Cl | 3-F | H | |
| 8.7 | $CH_3$ | Cl | 3-F,5-F | H | |
| 8.8 | $C_2H_5$ | Cl | n = 0 | H | |
| 8.9 | $C_2H_5$ | Cl | 3-$CF_3$ | H | |
| 8.10 | $C_2H_5$ | Cl | 3-Cl | H | |
| 8.11 | $C_2H_5$ | Cl | 3-F | H | |
| 8.12 | $C_2H_5$ | Cl | 3-F,5-F | H | |
| 8.13 | $C_3H_7$ | Cl | n = 0 | H | |
| 8.14 | $C_3H_7$ | Cl | 3-Cl | H | |
| 8.15 | $C_3H_7$ | Cl | 3-F | H | |
| 8.16 | cyclo-$C_3H_5$ | Cl | n = 0 | H | |
| 8.17 | cyclo-$C_3H_5$ | Cl | 3-Cl | H | |
| 8.18 | cyclo-$C_3H_5$ | Cl | 3-F | H | |
| 8.19 | i-$C_3H_7$ | Cl | n = 0 | H | |
| 8.20 | i-$C_3H_7$ | Cl | 3-Cl | H | |
| 8.21 | i-$C_3H_7$ | Cl | 3-F | H | |
| 8.22 | i-$C_4H_9$ | Cl | n = 0 | H | |
| 8.23 | i-$C_4H_9$ | Cl | 3-Cl | H | |
| 8.24 | i-$C_4H_9$ | Cl | 3-F | H | |
| 8.25 | $C_2H_5$ | Br | n = 0 | H | |
| 8.26 | $C_2H_5$ | Br | 3-Cl | H | |
| 8.27 | $C_2H_5$ | Br | 3-F | H | |
| 8.28 | $C_3H_7$ | Br | n = 0 | H | |

| Verb. Nr. | $R_1$ | $R_2$ | $(R_3)_n$ | $R_4$ | phys. Daten |
|---|---|---|---|---|---|
| 8.29 | $C_3H_7$ | Br | 3-Cl | H | |
| 8.30 | $C_3H_7$ | Br | 3-F | H | |

Beispiel H5: In analoger Weise wie in Beispiel H1 angegeben, gegebenenfalls nach zusätzlicher chromatographischer Reinigung [$CH_2Cl_2/(C_2H_5)_2O$ = 3:2], kann man auch die in der Tabelle 9 aufgeführte Verbindung der Formel II herstellen.

Tabelle 9

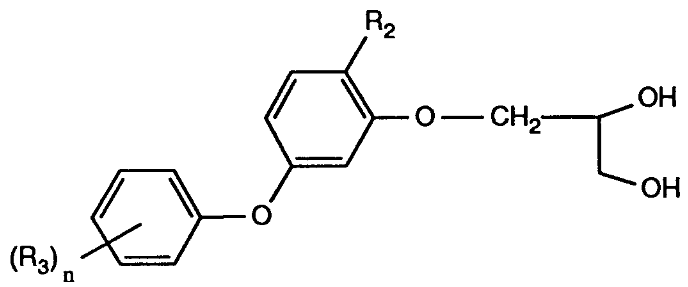

| Verb. Nr. | $R_2$ | $(R_3)_n$ | phys. Daten |
|---|---|---|---|
| 9.1 | Cl | 3-Cl | $n_D^{20} = 1.5909$ |

Formulierungsbeispiele

| Beispiel F1: Emulsions-Konzentrate | a) | b) | c) |
|---|---|---|---|
| Wirkstoff gemäss Herstellungsbeispielen | 25 % | 40 % | 50 % |
| Ca-Dodecylbenzolsulfonat | 5 % | 8 % | 6 % |
| Ricinusölpolyethylenglykolether (36 mol EO) | 5 % | - | - |
| Tributylphenolpolyethylenglykolether (30 mol EO) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| Beispiel F2: Lösungen | a) | b) | c) | d) |
|---|---|---|---|---|
| Wirkstoff gemäss Herstellungsbeispielen | 80 % | 10 % | 5 % | 95 % |
| Ethylenglykolmonomethylether | 20 % | - | - | - |
| Polyethylenglykol (MG 400) | - | 70 % | - | - |
| N-Methyl-2-pyrrolidon | - | 20 % | - | - |
| Epoxidiertes Kokosnussöl | - | - | 1 % | 5 % |
| Benzin (Siedegrenzen: 160-190°C) | - | - | 94 % | - |

Die Lösungen sind zur Anwendung in Form kleinster Tropfen geeignet.

| Beispiel F3: Granulate | a) | b) |
|---|---|---|
| Wirkstoff gemäss Herstellungsbeispielen | 5 % | 10 % |
| Kaolin | 94 % | - |
| Hochdisperse Kieselsäure | 1 % | - |
| Attapulgit | - | 90 % |

Der Wirkstoff wird in Dichlormethan gelöst, die Lösung auf den Träger aufgesprüht und das Lösungsmittel anschliessend im Vakuum abgedampft.

| Beispiel F4: Stäubemittel | a) | b) |
|---|---|---|
| Wirkstoff gemäss Herstellungsbeispielen | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

| Beispiel F5: Spritzpulver | a) | b) | c) |
|---|---|---|---|
| Wirkstoff gemäss Herstellungsbeispielen | 20 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyethylenglykolether (7-8 mol EO) | - | 2 % | - |
| Hochdisperse Kieselsäure | 5 % | 10 % | 10 % |
| Kaolin | 67 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| Beispiel F6: Emulsions-Konzentrat | a) | b) |
|---|---|---|
| Wirkstoff gemäss Herstellungsbeispielen | 10 % | 10 % |
| Octylphenolpolyethylenglykolether (4-5 mol EO) | 3 % | - |
| Ca-Dodecylbenzolsulfonat | 3 % | - |
| Ricinusölpolyglykolether (36 mol EO) | 4 % | - |
| Ricinusölthioxilat | - | 25 % |
| Cyclohexanon | 30 % | - |
| Butanol | - | 15 % |
| Xylolgemisch | 50 % | - |
| Essigsäureethylester | - | 50 % |

Aus diesen Konzentraten können durch Verdünnen mit Wasser Emulsionen jeder gewünschten Konzentration hergestellt werden.

| Beispiel F7: Stäubemittel | a) | b) |
|---|---|---|
| Wirkstoff gemäss Herstellungsbeispielen | 5 % | 8 % |
| Talkum | 95 % | - |
| Kaolin | - | 92 % |

Man erhält anwendungsfertige Stäubemittel, indem der Wirkstoff mit dem Träger vermischt und in einer geeigneten Mühle vermahlen wird.

| Beispiel F8: Extruder-Granulat | |
|---|---|
| Wirkstoff gemäss Herstellungsbeispielen | 10 % |
| Na-Ligninsulfonat | 2 % |
| Carboxymethylcellulose | 1 % |
| Kaolin | 87 % |

Der Wirkstoff wird mit den Zusatzstoffen vermischt und das Gemisch vermahlen und mit Wasser angefeuchtet. Dieses Gemisch wird extrudiert und anschließend im Luftstrom getrocknet.

| Beispiel F9: Umhüllungs-Granulat | |
|---|---|
| Wirkstoff gemäss Herstellungsbeispielen | 3 % |
| Polyethylenglykol (MG 200) | 3 % |
| Kaolin | 94 % |

Der feingemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

| Beispiel F10:Suspensions-Konzentrat | |
|---|---|
| Wirkstoff gemäss Herstellungsbeispielen | 40 % |
| Ethylenglykol | 10 % |
| Nonylphenolpolyethylenglykolether (15 mol EO) | 6 % |
| Na-Ligninsulfonat | 10 % |
| Carboxymethylcellulose | 1 % |
| 37%ige wässrige Formaldehyd-Lösung | 0,2% |
| Silikonöl in Form einer 75%igen wässrigen Emulsion | 0,8% |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Biologische Beispiele

Beispiel B1: Wirkung gegen Boophilus microplus

Vollgesogene adulte Zecken (Weibchen) werden auf eine PVC-Platte geklebt und mit einem Wattebausch überdeckt. Zur Behandlung werden die Testtiere mit 10 ml einer wässrigen Testlösung, die 125 ppm des zu prüfenden Wirkstoffes enthält, übergossen. Anschliessend wird der Wattebausch entfernt und die Zecken werden für 4 Wochen zur Eiablage inkubiert. Die Wirkung gegen Boophilus microplus zeigt sich entweder beim Weibchen als Mortalität oder Sterilität, oder bei den Eiern als ovizide Wirkung.
Verbindungen gemäss den Tabellen 2 bis 8 zeigen in diesem Test gute Wirkung.

Beispiel B2: Ovizide Wirkung auf Cydia pomonella

Auf Filterpapier abgelegte Eier von Cydia pomonella werden für eine kurze Zeit in eine acetonisch-wässrige Testlösung, die 400 ppm des zu prüfenden Wirkstoffes enthält, eingetaucht. Nach dem Antrocknen der Testlösung werden die Eier in Petrischalen inkubiert. Nach 6 Tagen wird der prozentuale Schlupf der Eier im Vergleich zu unbehandelten Kontrollansätzen ausgewertet (% Schlupfreduktion).
Verbindungen gemäss den Tabellen 2 bis 8 zeigen in diesem Test gute Wirkung.

Beispiel B3: Ovizide Wirkung auf Adoxophyes reticulana

Auf Filterpapier abgelegte Eier von Adoxophyes reticulana werden für kurze Zeit in eine acetonisch-wässrige Testlösung, die 400 ppm des zu prüfenden Wirkstoffes enthält, eingetaucht. Nach dem Antrocknen der Testlösung werden die Eier in Petrischalen inkubiert. Nach 6 Tagen wird der prozentuale Schlupf der Eier im Vergleich zu unbehandelten Kontrollansätzen ausgewertet (% Schlupfreduktion).
Verbindungen gemäss den Tabellen 2 bis 8 zeigen in diesem Test gute Wirkung.

Beispiel B4: Ovizide Wirkung auf Lobesia botrana

Auf Filterpapier abgelegte Eier von Lobesia botrana werden für kurze Zeit in eine acetonisch-wässrige Testlösung, die 400 ppm des zu prüfenden Wirkstoffes enthält, eingetaucht. Nach dem Antrocknen der Testlösung werden die Eier in Petrischalen inkubiert. Nach 6 Tagen wird der prozentuale Schlupf der Eier im Vergleich zu unbehandelten Kontrollansätzen ausgewertet (% Schlupfreduktion).
Verbindungen gemäss den Tabellen 2 bis 8 zeigen in diesem Test gute Wirkung.

Beispiel B5: Wirkung gegen Aonidiella aurantii

Kartoffelknollen werden mit Wanderlarven ("Crawlern") von Aonidiella aurantii (rote Citrus-Schildlaus) besiedelt. Nach etwa 2 Wochen werden die Kartoffeln in eine wässrige Emulsions- bzw. Suspensions- Spritzbrühe getaucht, die den zu prüfenden Wirkstoff in einer Konzentration von 400 ppm enthält. Nach dem Abtrocknen der so behandelten Kartoffelknollen werden diese in einem Plastikbehälter inkubiert. Zur Auswertung wird 10-12 Wochen später die Ueberlebensrate der Wanderlarven der ersten Folgegeneration der behandelten Schildlaus-Population mit derjenigen der unbehandelten Kontrollansätze verglichen.
Verbindungen gemäss den Tabellen 2 bis 8 zeigen in diesem Test gute Wirkung.

Beispiel B6: Wirkung gegen Nilaparvata lupens

Reispflanzen werden mit einer wässrigen Emulsions-Spritzbrühe, die 400 ppm des Wirkstoffes enthält, behandelt. Nach dem Antrocknen des Spritzbelages werden die Reispflanzen mit Zikadenlarven des 2. und 3. Stadiums besiedelt. 21 Tage später erfolgt die Auswertung. Aus dem Vergleich der Anzahl überlebender Zikaden auf den behandelten zu denjenigen auf den unbehandelten Pflanzen wird die prozentuale Reduktion der Population (% Wirkung) bestimmt.
Verbindungen gemäss den Tabellen 2 bis 8 zeigen in diesem Test gute Wirkung.

Beispiel B7: Wirkung gegen Nephotettix cincticeps

Reispflanzen werden mit einer wässrigen Emulsions-Spritzbrühe, die 400 ppm des Wirkstoffes enthält, behandelt. Nach dem Antrocknen des Spritzbelages werden die Reispflanzen mit Zikadenlarven des 2. und 3. Stadiums besiedelt. 21 Tage später erfolgt die Auswertung. Aus dem Vergleich der Anzahl überlebender Zikaden auf den behandelten zu denjenigen auf den unbehandelten Pflanzen wird die prozentuale Reduktion der Population (% Wirkung) bestimmt.
Verbindungen gemäss den Tabellen 2 bis 8 zeigen in diesem Test gute Wirkung.

Beispiel B8: Wirkung gegen Bemisia tabaci

Buschbohnen-Pflanzen werden in Gazekäfige gestellt und mit Adulten von Bemisia tabaci (Weisse Fliege) besiedelt. Nach erfolgter Eiablage werden alle Adulten entfernt und 10 Tage später die Pflanzen mit den darauf befindenden Nymphen mit einer wässrigen Emulsions-Spritzbrühe der zu prüfenden Wirkstoffe (Konzentration 400 ppm) behandelt. Die Auswertung erfolgt 14 Tage nach der Wirkstoff-Applikation auf %-Schlupf im Vergleich zu den unbehandelten Kontrollansätzen.
Verbindungen gemäss den Tabellen 2 bis 8 zeigen in diesem Test gute Wirkung.

Beispiel B9: Wirkung gegen Tetranychus urticae

Junge Bohnenpflanzen werden mit einer Mischpopulation von Tetranychus urticae besiedelt und 1 Tag später mit einer wässrigen Emulsions- Spritzbrühe, die 400 ppm des Wirkstoffes enthält, besprüht. Die Pflanzen werden anschliessend für 6 Tage bei 25°C inkubiert und danach ausgewertet. Aus dem Vergleich der Anzahl toter Eier, Larven und Adulten auf den behandelten zu denjenigen auf den unbehandelten Pflanzen wird die prozentuäle Reduktion der Population (% Wirkung) bestimmt.
Verbindungen gemäss den Tabellen 2 bis 8 zeigen in diesem Test gute Wirkung.

Beispiel B10: Ovo/larvizide Wirkung auf Heliothis virescens

Auf Baumwolle abgelegte Eier von Heliothis virescens werden mit einer wässrigen Emulsions-Spritzbrühe, die 400 ppm des Wirkstoffes enthält, besprüht. Nach 8 Tagen wird der prozentuale Schlupf der Eier und die Ueberlebensrate der Raupen im Vergleich zu unbehandelten Kontrollansätzen ausgewertet (% Reduktion der Population).
Verbindungen gemäss den Tabellen 2 bis 8 zeigen in diesem Test gute Wirkung.

Beispiel B11: Wirkung gegen Panonychus ulmi(OP und Carb. resistent)

Apfelsämlinge werden mit adulten Weibchen von Panonychus ulmi besiedelt. Nach sieben Tagen werden die infizierten Pflanzen mit einer wässrigen Emulsion-Spritzbrühe, enthaltend 400 ppm der zu prüfenden Verbindung, bis zur Tropfennässe besprüht und im Gewächshaus kultiviert. Nach 14 Tagen erfolgt die Auswertung. Aus dem Vergleich der Anzahl toter Spinnmilben auf den behandelten zu denjenigen auf den unbehandelten Pflanzen wird die prozentuale Reduktion an Population (% Wirkung) bestimmt.
Verbindungen gemäss den Tabellen 2 bis 8 zeigen in diesem Test gute Wirkung.

Beispiel B12: Wirkung gegen Ctenocephalides felis

20 bis 25 Floheier werden in eine waagrecht stehend 50-ml-Zellkulturflasche gegeben, in der zuvor 15 g Flohlarven-Nährmedium, welches 100 ppm des zu prüfenden Wirkstoffs enthält, vorgelegt wurde. Die Testflaschen werden in einem Brutschrank bei 26 bis 27°C und 60-70 % Luftfeuchtigkeit bebrütet. Nach 21 Tagen wird die Anwesenheit von adulten Flöhen, nicht geschlüpften Puppen und Larven überprüft.
Verbindungen gemäss den Tabellen 2 bis 8 zeigen in diesem Test gute Wirkung.

Beispiel B13: Wirkung gegen Bemisia tabaci Eier

Buschbohnen-Pflanzen werden in Gazekäfige gestellt und mit Adulten von Bemisia tabaci (Weisse Fliege) besiedelt. Nach erfolgter Eiablage werden alle Adulten entfernt und 2 Tage später die Pflanzen mit den darauf befindenden Nymphen mit einer wässrigen Emulsions-Spritzbrühe der zu prüfenden Wirkstoffe (Konzentration 400 ppm) behandelt. Die Auswertung erfolgt 10 Tage nach der Wirkstoff-Applikation auf %-Schlupf im Vergleich zu den unbehandelten Kontrollansätzen. Verbindungen gemäss den Tabellen 2 bis 8 zeigen in diesem Test gute Wirkung.

**Patentansprüche**

**1.** Eine Verbindung der Formel

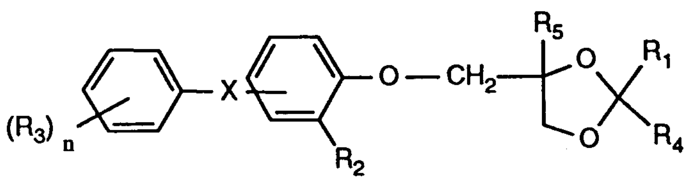

(I),

worin bedeuten:
n 0, 1 oder 2, wobei, wenn n 2 ist, die beiden Reste $R_3$ gleich oder verschieden sind;
entweder $R_1$ $C_1$-$C_6$-Alkyl, Halogen-$C_1$-$C_3$-alkyl, $C_2$-$C_4$-Alkenyl, $C_2$-$C_4$-Alkinyl, $C_1$-$C_3$-Alkoxy oder $C_3$-$C_6$-Cycloalkyl und $R_4$ Wasserstoff oder $C_1$-$C_3$-Alkyl oder $R_1$ und $R_4$, zusammen mit dem Kohlenstoffatom, an das $R_1$ und $R_4$ gebunden sind, einen Ring mit 4-, 5- oder 6-Ringgliedern, wobei das Ringgerüst, das gegebenenfalls eine Kohlenstoff-Kohlenstoff-Doppelbindung enthält, entweder nur aus Kohlenstoffatomen oder aus 1 Sauerstoffatom und 3, 4 oder 5 Kohlenstoffatomen aufgebaut ist und wobei der Ring unsubstituiert oder ein- oder zweifach durch gleiches oder verschiedenes $C_1$-$C_3$-Alkyl substituiert ist;
$R_2$ $C_1$-$C_3$-Alkyl, Halogen-$C_1$-$C_3$-alkyl, $C_1$-$C_3$-Alkoxy, Halogen-$C_1$-$C_3$-alkoxy, Fluor, Chlor oder Brom;
$R_3$ $C_1$-$C_3$-Alkyl, Halogen-$C_1$-$C_3$-alkyl, $C_1$-$C_3$-Alkoxy, Halogen-$C_1$-$C_3$-alkoxy, Fluor, Chlor oder Brom;
$R_5$ Wasserstoff oder $C_1$-$C_3$-Alkyl und
X Methylen, O, S oder C(=O).

**2.** Eine Verbindung gemäss Anspruch 1 der Formel

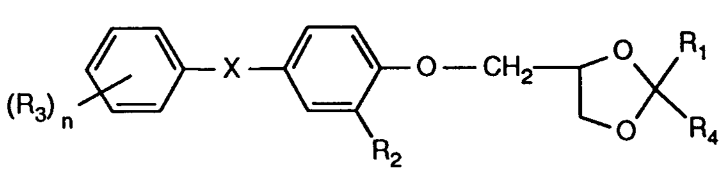

(Ia),

worin bedeuten:
n 0, 1 oder 2, wobei, wenn n 2 ist, die beiden Reste $R_3$ gleich oder verschieden sind;
$R_1$ $C_1$-$C_4$-Alkyl, Vinyl oder Cyclopropyl;
$R_2$ Methyl oder Chlor;
$R_3$ Methyl, Fluor oder Chlor;
$R_4$ Wasserstoff oder Methyl und
X Sauerstoff oder Methylen.

**3.** Eine Verbindung gemäss Anspruch 2 der Formel Ia, worin X Sauerstoff bedeutet.

**4.** Eine Verbindung gemäss Anspruch 2 oder 3 der Formel Ia, worin $R_2$ Chlor bedeutet.

**5.** Eine Verbindung gemäss einem der Ansprüche 2 bis 4 der Formel Ia, worin $R_4$ für Wasserstoff steht.

**6.** Eine Verbindung gemäss einem der Ansprüche 2 bis 5 der Formel Ia, worin $R_1$ $C_1$-$C_4$-Alkyl ist.

**7.** Eine Verbindung gemäss Anspruch 6 der Formel Ia, worin n 1 oder 2 und $R_3$ Fluor oder Chlor bedeuten.

**8.** Eine Verbindung gemäss Anspruch 7 der Formel Ia, worin der Rest $R_3$ oder einer der Reste $R_3$ in 3-Stellung gebunden ist.

**9.** Eine Verbindung gemäss Anspruch 2 der Formel Ia, worin X Methylen, $R_2$ Chlor und $R_4$ Wasserstoff bedeuten.

**10.** Eine Verbindung gemäss Anspruch 9 der Formel Ia, worin $R_1$ $C_1$-$C_4$-Alkyl ist.

**11.** Eine Verbindung gemäss Anspruch 10 der Formel Ia, worin n 1 oder 2 und $R_3$ Fluor oder Chlor bedeuten und der Rest $R_3$ oder einer der Reste $R_3$ in 3-Stellung gebunden ist.

**12.** Eine Verbindung gemäss Anspruch 2 der Formel Ia, ausgewählt aus der Gruppe von Verbindungen bestehend aus
4-(2-Chlor-4-phenoxy-phenoxymethyl)-2-ethyl-1,3-dioxolan;
4-(2-Chlor-4-phenoxy-phenoxymethyl)-2-propyl-1,3-dioxolan;
4-(2-Chlor-4-phenoxy-phenoxymethyl)-2-isopropyl-1,3-dioxolan;
4-(2-Chlor-4-phenoxy-phenoxymethyl)-2-cyclopropyl-1,3-dioxolan;
4-[2-Chlor-4-(3-fluorphenoxy)-phenoxymethyl]-2-ethyl-1,3-dioxolan;
4-[2-Chlor-4-(3-fluorphenoxy)-phenoxymethyl]-2-propyl-1,3-dioxolan;
4-[2-Chlor-4-(3-fluorphenoxy)-phenoxymethyl]-2-isopropyl-1,3-dioxolan;
4-[2-Chlor-4-(3-fluorphenoxy)-phenoxymethyl]-2-cyclopropyl-1,3-dioxolan;
4-[2-Chlor-4-(3-chlorphenoxy)-phenoxymethyl]-2-ethyl-1,3-dioxolan;
4-[2-Chlor-4-(3-chlorphenoxy)-phenoxymethyl]-2-propyl-1,3-dioxolan;
4-[2-Chlor-4-(3-chlorphenoxy)-phenoxymethyl]-2-isopropyl-1,3-dioxolan;
4-[2-Chlor-4-(3,5-difluorphenoxy)-phenoxymethyl]-2-ethyl-1,3-dioxolan;
4-[2-Chlor-4-(3,5-difluorphenoxy)-phenoxymethyl]-2-propyl-1,3-dioxolan;
4-[2-Chlor-4-(3,5-difluorphenoxy)-phenoxymethyl]-2-isopropyl-1,3-dioxolan;
4-[2-Chlor-4-(3,4-dichlorphenoxy)-phenoxymethyl]-2-ethyl-1,3-dioxolan und
4-[2-Chlor-4-(3-chlor-4-fluor-phenoxy)-phenoxymethyl]-2-ethyl-1,3-dioxolan.

**13.** Eine Verbindung gemäss Anspruch 2 der Formel Ia, ausgewählt aus der Gruppe von Verbindungen bestehend aus
4-(2-Chlor-4-benzyl-phenoxymethyl)-2-ethyl-1,3-dioxolan;
4-(2-Chlor-4-benzyl-phenoxymethyl)-2-propyl-1,3-dioxolan;
4-(2-Chlor-4-benzyl-phenoxymethyl)-2-isopropyl-1,3-dioxolan;
4-(2-Chlor-4-benzyl-phenoxymethyl)-2-cyclopropyl-1,3-dioxolan;
4-[2-Chlor-4-(3-fluorbenzyl)-phenoxymethyl]-2-ethyl-1,3-dioxolan;
4-[2-Chlor-4-(3-fluorbenzyl)-phenoxymethyl]-2-propyl-1,3-dioxolan;
4-[2-Chlor-4-(3,5-difluorbenzyl)-phenoxymethyl]-2-ethyl-1,3-dioxolan und
4-[2-Chlor-4-(3,4-dichlorbenzyl)-phenoxymethyl]-2-ethyl-1,3-dioxolan.

**14.** Verfahren zur Herstellung einer Verbindung gemäss Anspruch 1 der Formel I, dadurch gekennzeichnet, dass man

a) eine Verbindung der Formel

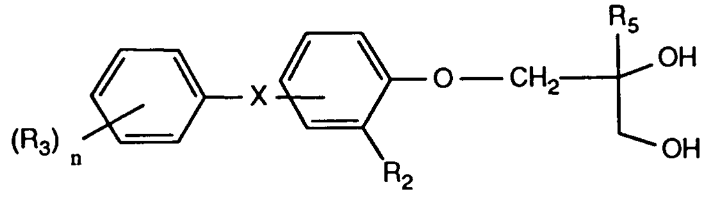

(II)

mit einer Verbindung der Formel

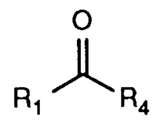

(III),

vorzugsweise in Gegenwart eines sauren Katalysators, umsetzt oder
b) eine Verbindung der Formel

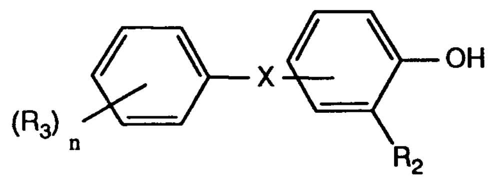

(IV)

mit einer Verbindung der Formel

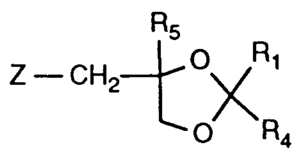

(V),

vorzugsweise in Gegenwart einer Base, umsetzt oder
c) eine Verbindung der Formel II mit einer Verbindung der Formel

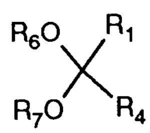

(VI),

vorzugsweise in Gegenwart eines sauren Katalysators, umsetzt, wobei n, $R_1$, $R_2$, $R_3$, $R_4$, $R_5$ und X jeweils die für die Formel I angegebenen Bedeutungen haben, $R_6$ und $R_7$ entweder, unabhängig voneinander, $C_1$-$C_8$-Alkyl oder zusammen $-(CH_2)_2$-oder $-(CH_2)_3$-bedeuten und Z Chlor, Brom, Iod, Methansulfonyloxy oder p-Toluolsulfonyloxy bedeutet und jeweils, wenn erwünscht, ein verfahrensgemäss erhältliches Gemisch von Isomeren auftrennt und das gewünschte Isomere isoliert.

**15.** Schädlingsbekämpfungsmittel, dadurch gekennzeichnet, dass es mindestens eine Verbindung gemäss Anspruch 1 als Wirkstoff und mindestens einen Hilfsstoff enthält.

**16.** Mittel gemäss Anspruch 15 zum Schutz von Pflanzen oder Tieren gegen den Befall durch Insekten und/oder Schädlinge der Ordnung Akarina.

**17.** Verfahren zur Herstellung eines Mittels gemäss Anspruch 15, dadurch gekennzeichnet, dass man den Wirkstoff mit dem (den) Hilfsstoff(en) innig vermischt.

**18.** Verwendung einer Verbindung gemäss Anspruch 1 oder eines Mittels gemäss Anspruch 15 zur Bekämpfung von Schädlingen.

**19.** Verwendung gemäss Anspruch 18 zum Schutz von Pflanzen oder Tieren gegen den Befall durch Insekten und/oder Schädlinge der Ordnung Akarina.

**20.** Verwendung gemäss Anspruch 19 zur Bekämpfung von pflanzenschädigenden saugenden Insekten.

**21.** Verwendung gemäss Anspruch 19 zur Bekämpfung von Schildläusen oder weissen Fliegen.

**22.** Verwendung gemäss Anspruch 19 zur Bekämpfung larvaler Stadien pflanzenschädigender Insekten.

**23.** Verwendung gemäss Anspruch 18 zur Behandlung von Saatgut.

**24.** Verfahren zur Bekämpfung von Schädlingen, dadurch gekennzeichnet, dass man die Schädlinge beziehungsweise deren verschiedene Entwicklungsstadien oder ihren Aufenthaltsort mit einer pestizid wirksamen Menge einer Verbindung gemäss Anspruch 1 oder einem Mittel gemäss Anspruch 15 behandelt.

**25.** Verfahren gemäss Anspruch 24 zum Schutz von Pflanzen oder Tieren gegen den Befall durch Insekten und/oder Schädlinge der Ordnung Akarina.

**26.** Verfahren gemäss Anspruch 24 zum Schutz von Saatgut, dadurch gekennzeichnet, dass man das Saatgut oder die Saatfurche behandelt.

**27.** Saatgut, behandelt gemäss dem in Anspruch 26 beschriebenen Verfahren.

**28.** Eine Verbindung der Formel

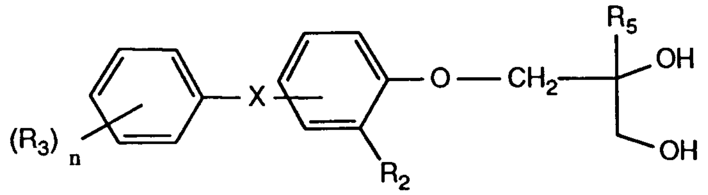

(II),

worin n, $R_2$, $R_3$, $R_5$ und X die für die Formel I angegebenen Bedeutungen haben.

## Claims

**1.** A compound of the formula

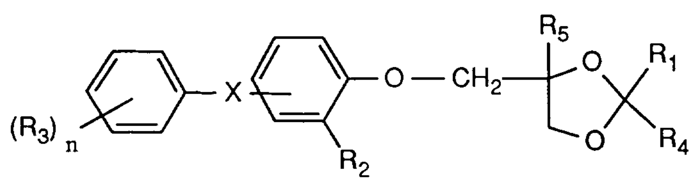

(I)

in which:
n is 0, 1 or 2, and, if n is 2, the two radicals $R_3$ are identical or different;
either $R_1$ is $C_1$-$C_6$alkyl, halo-$C_1$-$C_3$alkyl, $C_2$-$C_4$alkenyl, $C_2$-$C_4$alkynyl, $C_1$-$C_3$alkoxy or $C_3$-$C_6$cycloalkyl and $R_4$ is hydrogen or $C_1$-$C_3$alkyl, or $R_1$ and $R_4$ together with the carbon atom to which $R_1$ and $R_4$ are bonded are a ring having 4, 5 or 6 ring members, and the ring skeleton, which may contain a carbon-carbon double bond, is composed either only of carbon atoms or of 1 oxygen atom and 3, 4 or 5 carbon atoms, and the ring is unsubstituted or mono- or disubstituted by identical or different $C_1$-$C_3$alkyl radicals;
$R_2$ is $C_1$-$C_3$alkyl, halo-$C_1$-$C_3$alkyl, $C_1$-$C_3$alkoxy, halo-$C_1$-$C_3$alkoxy, fluorine, chlorine or bromine;
$R_3$ is $C_1$-$C_3$alkyl, halo-$C_1$-$C_3$alkyl, $C_1$-$C_3$alkoxy, halo-$C_1$-$C_3$alkoxy, fluorine, chlorine or bromine;
$R_5$ is hydrogen or $C_1$-$C_3$alkyl and
X is methylene, O, S or C(=O).

2. A compound according to claim 1 of the formula

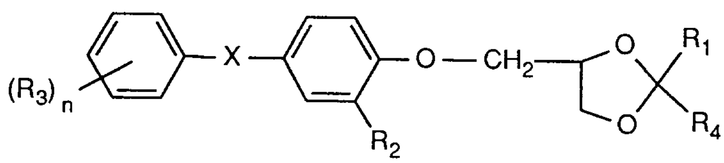

(Ia)

in which:
n is 0, 1 or 2, and, if n is 2, the two radicals $R_3$ are identical or different;
$R_1$ is $C_1$-$C_4$alkyl, vinyl or cyclopropyl;
$R_2$ is methyl or chlorine;
$R_3$ is methyl, fluorine or chlorine;
$R_4$ is hydrogen or methyl and
X is oxygen or methylene.

3. A compound according to claim 2 of the formula Ia in which X is oxygen.

4. A compound according to claim 2 or 3 of the formula Ia in which $R_2$ is chlorine.

5. A compound according to any one of claims 2 to 4 of the formula Ia in which $R_4$ is hydrogen.

6. A compound according to any one of claims 2 to 5 of the formula Ia in which $R_1$ is $C_1$-$C_4$alkyl.

7. A compound according to claim 6 of the formula Ia in which n is 1 or 2 and $R_3$ is fluorine or chlorine.

8. A compound according to claim 7 of the formula Ia in which the radical $R_3$ or one of the radicals $R_3$ is bonded in the 3-position.

9. A compound according to claim 2 of the formula Ia in which X is methylene, $R_2$ is chlorine and $R_4$ is hydrogen.

10. A compound according to claim 9 of the formula Ia in which $R_1$ is $C_1$-$C_4$alkyl.

11. A compound according to claim 10 of the formula Ia in which n is 1 or 2 and $R_3$ is fluorine or chlorine and the radical $R_3$ or one of the radicals $R_3$ is bonded in the 3-position.

12. A compound according to claim 2 of the formula Ia, selected from the group of compounds comprising
4-(2-chloro-4-phenoxyphenoxymethyl)-2-ethyl-1,3-dioxolane;
4-(2-chloro-4-phenoxyphenoxymethyl)-2-propyl-1,3-dioxolane;
4-(2-chloro-4-phenoxyphenoxymethyl)-2-isopropyl-1,3-dioxolane;
4-(2-chloro-4-phenoxyphenoxymethyl)-2-cyclopropyl-1,3-dioxolane;
4-[2-chloro-4-(3-fluorophenoxy)phenoxymethyl]-2-ethyl-1,3-dioxolane;
4-[2-chloro-4-(3-fluorophenoxy)phenoxymethyl]-2-propyl-1,3-dioxolane;
4-[2-chloro-4-(3-fluorophenoxy)phenoxymethyl]-2-isopropyl-1,3-dioxolane;
4-[2-chloro-4-(3-fluorophenoxy)phenoxymethyl]-2-cyclopropyl-1,3-dioxolane;
4-[2-chloro-4-(3-chlorophenoxy)phenoxymethyl]-2-ethyl-1,3-dioxolane;
4-[2-chloro-4-(3-chlorophenoxy)phenoxymethyl]-2-propyl-1,3-dioxolane;
4-[2-chloro-4-(3-chlorophenoxy)phenoxymethyl]-2-isopropyl-1,3-dioxolane;
4-[2-chloro-4-(3,5-difluorophenoxy)phenoxymethyl]-2-ethyl-1,3-dioxolane;
4-[2-chloro-4-(3,5-difluorophenoxy)phenoxymethyl]-2-propyl-1,3-dioxolane;
4-[2-chloro-4-(3,5-difluorophenoxy)phenoxymethyl]-2-isopropyl-1,3-dioxolane;
4-[2-chloro-4-(3,4-dichlorophenoxy)phenoxymethyl]-2-ethyl-1,3-dioxolane and
4-[2-chloro-4-(3-chloro-4-fluorophenoxy)phenoxymethyl]-2-ethyl-1,3-dioxolane.

13. A compound according to claim 2 of the formula Ia, selected from the group of compounds comprising
4-(2-chloro-4-benzylphenoxymethyl)-2-ethyl-1,3-dioxolane;
4-(2-chloro-4-benzylphenoxymethyl)-2-propyl-1,3-dioxolane;

4-(2-chloro-4-benzylphenoxymethyl)-2-isopropyl-1,3-dioxolane;
4-(2-chloro-4-benzylphenoxymethyl)-2-cyclopropyl-1,3-dioxolane;
4-[2-chloro-4-(3-fluorobenzyl)phenoxymethyl]-2-ethyl-1,3-dioxolane;
4-[2-chloro-4-(3-fluorobenzyl)phenoxymethyl]-2-propyl-1,3-dioxolane;
4-[2-chloro-4-(3,5-difluorobenzyl)phenoxymethyl]-2-ethyl-1,3-dioxolane and
4-[2-chloro-4-(3,4-dichlorobenzyl)phenoxymethyl]-2-ethyl-1,3-dioxolane.

**14.** A process for the preparation of a compound according to claim 1 of the formula I, which comprises

a) reacting a compound of the formula

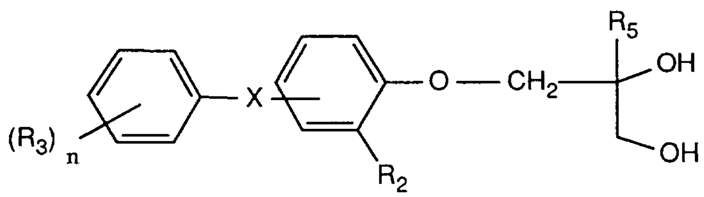

(II)

with a compound of the formula

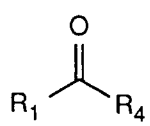

(III),

preferably in the presence of an acidic catalyst, or

b) reacting a compound of the formula

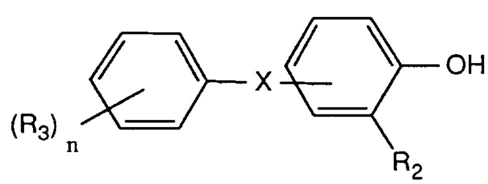

(IV)

with a compound of the formula

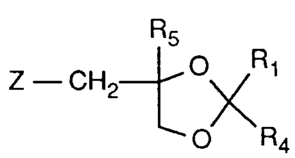

(V),

preferably in the presence of a base, or

c) reacting a compound of the formula II with a compound of the formula

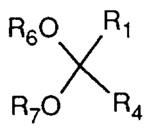

(VI),

preferably in the presence of an acidic catalyst, where n, $R_1$, $R_2$, $R_3$, $R_4$, $R_5$ and X are in each case as defined for formula I, $R_6$ and $R_7$ either independently of one another are $C_1$-$C_8$alkyl or together are $-(CH_2)_2-$ or $-(CH_2)_3-$ and Z is chlorine, bromine, iodine, methanesulfonyloxy or p-toluenesulfonyloxy and, if desired, in each case a mixture of isomers which can be obtained according to the process is separated and the desired isomer is isolated.

**15.** A pesticide, which comprises at least one compound according to claim 1 as active ingredient and at least one adjuvant.

**16.** A composition according to claim 15 for protecting plants or animals against infestation with insects and/or pests from the order of the Acarina.

**17.** A process for the preparation of a composition according to claim 15, which comprises intimately mixing the active ingredient with the adjuvant(s).

**18.** The use of a compound according to claim 1, or of a composition according to claim 15, for controlling pests.

**19.** The use according to claim 18 for protecting plants or animals against infestation with insects and/or pests from the order of the Acarina.

**20.** The use according to claim 19 for controlling plant-injurious sucking insects.

**21.** The use according to claim 19 for controlling scale insects or whitefly.

**22.** The use according to claim 19 for controlling larval stages of plant-injurious insects.

**23.** The use according to claim 18 for the treatment of seed.

**24.** A method of controlling pests, which comprises treating the pests or the various development stages of the pests or their locus with a pesticidally effective amount of a compound according to claim 1 or a composition according to claim 15.

**25.** A method according to claim 24 for protecting plants or animals against infestation with insects and/or pests from the order of the Acarina.

**26.** A method according to claim 24 for protecting seed, which comprises treating the seed or the seed furrow.

**27.** Seed treated according to the method described in claim 26.

**28.** A compound of the formula

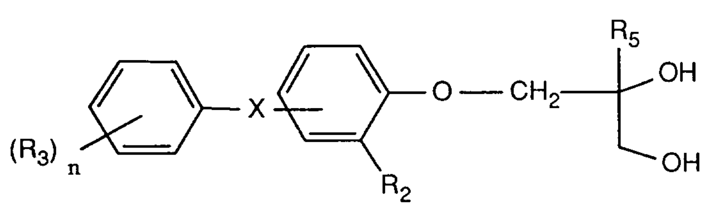

(II)

in which n, $R_2$, $R_3$, $R_5$ and X are as defined for formula I.

**Revendications**

1. Composé de formule

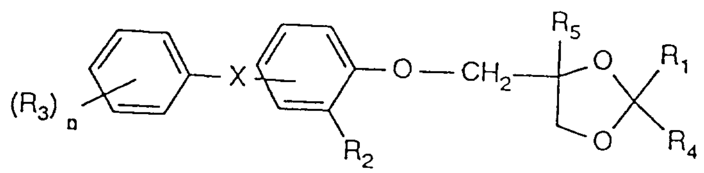

(I),

où :
n vaut 0, 1 ou 2, où quand n est égal à 2, les deux restes $R_3$ sont identiques ou différents ; soit $R_1$ représente un alkyle $C_{1-6}$, un alkyle $C_{1-3}$ halogéné, un alcényle $C_{2-4}$, un alcynyle $C_{2-4}$ , un alcoxy $C_{1-3}$ soit un cycloalkyle $C_{3-6}$ et $R_4$ représente l'hydrogène ou un alkyle $C_{1-3}$ ou $R_1$ et $R_4$, sont liés ensemble avec l'atome de carbone auquel $R_1$ et $R_4$ sont fixés pour donner un cycle de 4, 5 ou 6 maillons, ou la structure de cycle, qui contient le cas échéant une double liaison carbone-carbone, est constituée soit de seulement des atomes de carbone soit d'un atome d'oxygène et de trois, quatre ou cinq atomes de carbone, et où le cycle est non substitué ou est substitué une ou deux fois par un alkyle $C_{1-3}$ identique ou différent ;
$R_2$ représente un alkyle $C_{1-3}$ , un alkyle $C_{1-3}$ halogéné, un alcoxy $C_{1-3}$ , un alcoxy $C_{1-3}$ halogéné, le fluor, le chlore ou le brome ;
$R_3$ représente un alkyle $C_{1-3}$ , un alkyle $C_{1-3}$ halogéné, un alcoxy $C_{1-3}$, un alcoxy $C_{1-3}$ halogéné, le fluor, le chlore ou le brome ;
$R_5$ représente l'hydrogène ou un alyyle $C_{1-3}$ et
X représente le méthylène, O, S ou C(=O).

2. Composé selon la revendication 1, de formule

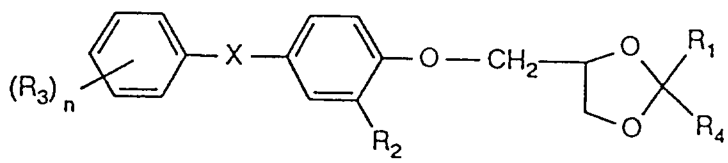

(Ia),

où :
n représente 0, 1 ou 2, où, quand n est 2, les deux restes $R_3$ sont identiques ou différents ;
$R_1$ représente un alkyle $C_{1-4}$, vinyle ou cyclopropyle ;
$R_2$ est un méthyle ou le chlore ;
$R_3$ est un méthyle, fluor ou chlore ;
$R_4$ est un hydrogène ou méthyle, et
X est un oxygène ou méthylène.

3. Composé selon la revendication 2 de formule Ia, où X représente l'oxygène.

4. Composé selon les revendications 2 ou 3 de formule Ia, où $R_2$ représente le chlore.

5. Composé selon l'une des revendications 2 à 4 de formule Ia, où $R_4$ représente l'hydrogène.

6. Composé selon l'une des revendications 2 à 5 de formule Ia, où $R_1$ est un alkyle $C_{1-4}$.

7. Composé selon la revendication 6 de formule Ia, où n représente 1 ou 2 et $R_3$ représente le fluor ou le chlore.

8. Composé selon la revendication 7 de formule Ia, où le reste $R_3$ ou un des restes $R_3$ est fixé en position 3.

**9.** Composé selon la revendication 2 de formule Ia, où X représente un méthylène, $R_2$ le chlore et $R_4$ l'hydrogène.

**10.** Composé selon la revendication 9 de formule Ia, où $R_1$ est un alkyle $C_{1-4}$.

**11.** Composé selon la revendication 10 de formule Ia, où n est 1 ou 2 et $R_3$ est le fluor ou le chlore et le reste $R_3$ ou l'un des restes $R_3$ est fixé en position 3.

**12.** Composé selon la revendication 2 de formule Ia, choisi dans le groupe des composés constitué de
4-(2-chloro-4-phénoxy-phénoxyméthyl)-2-éthyl-1,3-dioxolane ;
4-(2-chloro-4-phénoxy-phénoxyméthyl)-2-propyl-1,3-dioxolane ;
4-(2-chloro-4-phénoxy-phénoxyméthyl)-2-isopropyl-1,3-dioxolane ;
4-(2-chloro-4-phénoxy-phénoxyméthyl)-2-cyclopropyl-1,3-dioxolane ;
4-[2-chloro-4-(3-fluorophénoxy)-phénoxyméthyl]-2-éthyl-1,3-dioxolane ;
4-[2-chloro-4-(3-fluorophénoxy)-phénoxyméthyl]-2-propyl-1,3-dioxolane ;
4-[2-chloro-4-(3-fluorophénoxy)-phénoxyméthyl]-2-isopropyl-1,3-dioxolane ;
4-[2-chloro-4-(3-fluorophénoxy)-phénoxyméthyl]-2-cyclopropyl-1,3-dioxolane ;
4-[2-chloro-4-(3-chlorophénoxy)-phénoxyméthyl]-2-éthyl-1,3-dioxolane ;
4-[2-chloro-4-(3-chlorophénoxy)-phénoxyméthyl]-2-propyl-1,3-dioxolane ;
4-[2-chloro-4-(3-chlorophénoxy)-phénoxyméthyl]-2-isopropyl-1,3-dioxolane ;
4-[2-chloro-4-(3,5-difluorophénoxy)-phénoxyméthyl]-2-éthyl-1,3-dioxolane ;
4-[2-chloro-4-(3,5-difluorophénoxy)-phénoxyméthyl]-2-propyl-1,3-dioxolane ;
4-[2-chloro-4-(3,5-difluorophénoxy)-phénoxyméthyl]-2-isopropyl-1,3-dioxolane ;
4-[2-chloro-4-(3,4-dichlorophénoxy)-phénoxyméthyl]-2-éthyl-1,3-dioxolane ; et
4-[2-chloro-4-(3-chloro-4-fluorophénoxy)-phénoxyméthyl]-2-éthyl-1,3-dioxolane.

**13.** Composé selon la revendication 2 de formule Ia, choisi dans le groupe des composés constitués de
4-(2-chloro-4-benzyl-phénoxyméthyl)-2-éthyl-1,3-dioxolane ;
4-(2-chloro-4-benzyl-phénoxyméthyl)-2-propyl-1,3-dioxolane ;
4-(2-chloro-4-benzyl-phénoxyméthyl)-2-isopropyl-1,3-dioxolane ;
4-(2-chloro-4-benzyl-phénoxyméthyl)-2-cyclopropyl-1,3-dioxolane ;
4-[2-chloro-4-(3-fluorobenzyl)-phénoxyméthyl]-2-éthyl-1,3-dioxolane ;
4-[2-chloro-4-(3-fluorobenzyl)-phénoxyméthyl]-2-propyl-1,3-dioxolane ;
4-[2-chloro-4-(3,5-difluorobenzyl)-phénoxyméthyl]-2-éthyl-1,3-dioxolane ; et
4-[2-chloro-4-(3,4-dichlorobenzyl)-phénoxyméthyl]-2-éthyl-1,3-dioxolane.

**14.** Procédé de préparation d'un composé selon la revendication 1 de formule I, caractérisé en ce que

a) on fait réagir un composé de formule

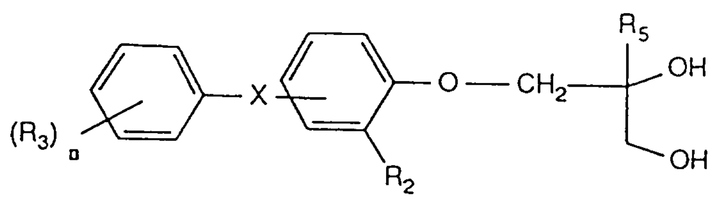

(II)

avec un composé de formule

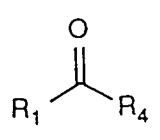

(III),

de préférence en présence d'un catalyseur acide, ou

b) on fait réagir un composé de formulre

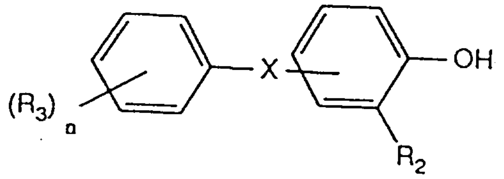

(IV)

avec un composé de formule

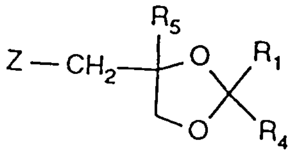

(V),

de préférence en présence d'une base, ou

c) on fait réagir un composé de formule II avec un composé de formule

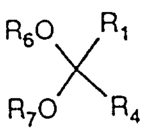

(VI),

de préférence en présence d'un catalyseur acide, où n, $R_1$, $R_2$, $R_3$, $R_4$, $R_5$ et $R_6$ ont respectivement les significations données pour la formule I, $R_6$ et $R_7$ soit, indépendamment l'un de l'autre représentent un alkyle $C_{1-8}$, soit ensemble $-(CH_2)_2-$ ou $-(CH_2)_3-$ et Z représente un chlore, brome, iode, méthanesulfonyloxy ou p-toluènesulfonyloxy, et respectivement, si c'est souhaité, on recueille un mélange des isomères obtenus selon le procédé et on isole l'isomère souhaité.

**15.** Moyen de lutte contre les parasites, caractérisé en ce qu'il contient au moins un compose selon la revendication 1 comme principe actif et au moins un produit auxiliaire.

**16.** Moyen selon la revendication 15 pour protéger des plantes et des animaux contre l'agression des insectes et/ou parasites de l'ordre Akarina.

**17.** Procédé de préparation d'un moyen selon la revendication 15, caractérisé en ce qu'on mélange intimement le principe actif avec le ou les produits auxiliaires.

**18.** Utilisation d'un composé selon la revendication 1 ou un moyen selon la revendication 15 pour combattre les parasites.

**19.** Utilisation selon la revendication 18 pour protéger des plantes ou des animaux de l'attaque par des insectes et/ou des parasites de l'ordre Akarina.

**20.** Utilisation selon la revendication 19 pour combattre les insectes suceurs endommageant les plantes.

**21.** Utilisation selon la revendication 19 pour combattre des cochenilles ou des mouches blanches.

**22.** Utilisation selon la revendication 19, pour combattre les stades larvaires des insectes endommageant les plantes.

**23.** Utilisation selon la revendication 18 pour traiter les semences.

**24.** Procédé de lutte contre les parasites, caractérisé en ce qu'on traite les parasites ou leurs différents stades de développement ou leur lieu de séjour avec une quantité efficace de pesticide d'un composé selon la revendication 1, ou d'un moyen selon la revendication 15.

**25.** Procédé selon la revendication 24 de protection des plantes ou des animaux contre la contamination par des insectes et/ou des parasites de l'ordre Akarina.

**26.** Procédé selon la revendication 24 pour protéger des semences, caractérisé en ce qu'on traite la semence ou le sillon de semis.

**27.** Semence traitée selon le procédé décrit à la revendication 26.

**28.** Composé de formule

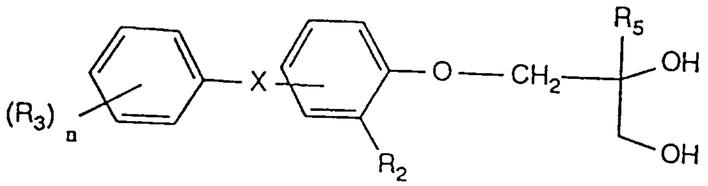

(II),

où n, $R_2$, $R_3$, $R_5$ et X ont les significations données pour la formule I.